(19) **Europäisches Patentamt**
**European Patent Office**
**Office européen des brevets**

(11) **EP 2 186 892 A1**

(12) **EUROPEAN PATENT APPLICATION**
published in accordance with Art. 153(4) EPC

(43) Date of publication:
**19.05.2010 Bulletin 2010/20**

(51) Int Cl.:
*C12N 15/09* (2006.01)　　*A61K 39/395* (2006.01)
*A61P 35/00* (2006.01)　　*A61P 37/02* (2006.01)
*C07K 16/28* (2006.01)　　*C12P 21/08* (2006.01)
*C12N 5/10* (2006.01)

(21) Application number: **07806885.5**

(22) Date of filing: **06.09.2007**

(86) International application number:
**PCT/JP2007/067444**

(87) International publication number:
**WO 2009/031230 (12.03.2009 Gazette 2009/11)**

(84) Designated Contracting States:
**AT BE BG CH CY CZ DE DK EE ES FI FR GB GR
HU IE IS IT LI LT LU LV MC MT NL PL PT RO SE
SI SK TR**
Designated Extension States:
**AL BA HR MK RS**

(71) Applicant: **Osaka University
Suita-shi
Osaka 565-0871 (JP)**

(72) Inventors:
• **UCHIYAMA, Susumu
Suita-shi
Osaka 565-0871 (JP)**
• **FUKUI, Kiichi
Suita-shi
Osaka 565-0871 (JP)**

(74) Representative: **Müller, Christian Stefan Gerd et al
Dr. Volker Vossius
Patent- und Rechtsanwaltskanzlei
Geibelstrasse 6
81679 München (DE)**

(54) **ANTI-CD20 MONOCLONAL ANTIBODIES**

(57) It is intended to provide a monoclonal antibody having a growth inhibitory activity against a cell having a human CD20 antigen which is produced by using, as immunogens, a human B cell line expressing the human CD20 antigen and a cell line originating in a non-human animal, which is different from an animal to be immunized and has been transformed with human CD20 DNA, and a monoclonal antibody obtained by chimerization or humanization of the above-described monoclonal antibody. These monoclonal antibodies show biological activities suitable for using as drugs.

**EP 2 186 892 A1**

**Description**

TECHNICAL FIELD

The present invention relates to an anti-CD20 monoclonal antibody.

BACKGROUND ART

CD20 is a protein not containing sugar chains, which is expressed on the cellular surface of human B-lymphocytes. CD20 is expressed on many malignant tumor B-cells, in addition to normal B-cells in the peripheral blood, spleen, tonsils, and bone marrow. Epitopes to which monoclonal antibodies directed to CD20 bind are extremely highly varied and a wide variety of biological responses have been reported. Furthermore, there have been many reports of monoclonal antibodies recognizing CD20. Among them, rituximab is a chimerized mouse/human monoclonal antibody (C2B8), which is derived from a mouse antibody 2B8 obtained by immunization of an SB cell strain, a type of human B-cell (see Patent Documents 1 and 2). Rituximab has been actually used under the name Rituxan® as a therapeutic agent for the treatment of low malignant non-Hodgkin's lymphoma (NHL). Since then, it has been further reported that Rituxan is effective against many immune diseases related to B-cells, for example, malignant tumors, such as chronic lymphocytic leukemia (CCL), autoimmune diseases involving pathogenic autoantibodies, such as autoimmune hemolyticanemia and idiopathic thrombocytopenic purpura (ITP), and inflammatory diseases, such as rheumatoid arthritis (RA) and multiple sclerosis (see Non-Patent Documents 1 to 4).
It has been reported that rituximab binds to human complements, resulting in lysis of B-cells of the lymphatic linenage by complement-dependent cytotoxicity (CDC) (see Non-Patent Document 5), and that rituximab displays activity in assays for antibody-dependent cell-mediated cytotoxicity (ADCC) and growth inhibiting activity and apoptosis induction in tritiated thymidine incorporation assays (see Non-Patent Document 6).
Chimera molecules with molecules from different animal species are antigenic and therefore are generally not desirable as therapeutic agents, while it has been believed that anti-CD20 antibodies, including rituximab, are not antigenic since they target and eliminate all types of B-cells, including normal cells. However, it has been reported that there are cases where rituximab induces neutralizing antibodies, albeit at several percent, during therapy, and the possibility of inducing neutralizing antibodies may further increase depending on its dosage amount and duration. In addition, there is an increased focus on problems associated with antigenicity, in the course of extending the target to be treated from B-cell lymphomas to RA, IT, and MS. For these reasons, there has been a recent need for human antibodies or humanized antibodies containing sequences more similar to human sequences.
Chimerized antibodies entail the problem that they have relatively short half-lives in blood. The $\beta$ half-life ($\beta$ 1/2) of chimerized mouse/human antibodies, including rituximab, is no more than 3 to 4 days. The efficacy in clinical trials of rituximab against low malignant NHL has been reported to be a little less than 50% (see Non-Patent Document 7). Furthermore, there is a problem that amounts of rituximab to be administered in NHL therapy are increased, since the dissociation constant (Kd value) of rituximab for the CD20 antigen is 5.2 nM and the binding affinity is not very high (see Non-Patent Document 8).

[Patent Document 1]: International Publication No. WO 94/11026 pamphlet
[Patent Document 2]: US Patent No. 5,736,137 specification [Non-Patent Document 1]: Coiffier B et al., Blood 1998; 92:1297-32
[Non-Patent Document 2]: Edward JC et al., Rheumatology (Oxford) 2001; 40:205-11
[Non-Patent Document 3]: Zaja F et al., Heamatologica 2002; 87:189-95
[Non-Patent Document 4]: Perrotta S et al., Br J Haematol 2002; 116:465-7
[Non-Patent Document 5]: Reff et al., Blood 1994; 83: 435-445
[Non-Patent Document 6]: Maloney et al., Blood 1996; 88: 637a
[Non-Patent Document 7]: IDEC Pharmaceuticals Corporation News Release, December 8, 1998
[Non-Patent Document 8]: Mitchell ER et al., Blood 1994; 82:435-445

DISCLOSURE OF THE INVENTION

PROBLEMS TO BE SOLVED BY THE INVENTION

In view of the problems discussed above, the present invention has the principal object of providing an anti-CD20 monoclonal antibody displaying biological activities more suitable as pharmaceutical use.

MEANS TO SOLVE THE PROBLEMS

In order to attain the above object, the present inventors have performed diligent research to prepare a monoclonal antibody displaying high binding affinity to human CD20 molecule in its natural state, thereby obtaining an anti-CD20 monoclonal antibody having excellent characteristics. In consequence, the inventors have found that high-affinity monoclonal antibodies displaying excellent biological activities can be obtained by using, as immunogens, SB or Raji cells, which are of a B-cell strain thought to contain a high density of CD20 antigen, combined with non-human animal cells modified using genetic recombination to express a large amount of CD20 on the cellular membrane, thereby having arrived at the present invention.

In other words, the present invention provides the following:

(1) a humanized anti-CD20 monoclonal antibody, comprising a combination of the L-chain set forth in any one of SEQ ID Nos: 27 to 30 and the H-chain set forth in any one of SEQ ID Nos: 31 to 34;
(2) the humanized anti-CD20 monoclonal antibody according to the above-described (1), comprising a combination of the L-chain set forth in SEQ ID No: 30 and the H-chain set forth in SEQ ID No: 34;
(3) the humanized anti-CD20 monoclonal antibody according to the above-described (1), comprising a combination of the L-chain set forth in SEQ ID No: 30 and the H-chain set forth in SEQ ID No: 31;
(4) the humanized anti-CD20 monoclonal antibody according to the above-described (1), comprising a combination of the L-chain set forth in SEQ ID No: 29 and the H-chain set forth in SEQ ID No: 33;
(5) the humanized anti-CD20 monoclonal antibody according to the above-described (1), which is produced by a cell selected from the group consisting of cells which have been deposited under the accession numbers FERM ABP-10907, FERM ABP-10906, and FERM ABP-10905, with the International Patent Organism Depositary of the National Institute of Advanced Industrial Science and Technology;
(6) a method for producing a humanized anti-CD20 monoclonal antibody, which comprises culturing a cell selected from the group consisting of cells which have been deposited under the accession numbers FERM ABP-10907, FERM ABP-10906, and FERM ABP-10905, with the International Patent Organism Depositary of the National Institute of Advanced Industrial Science and Technology;
(7) a therapeutic agent for the treatment of B-cell mediated diseases, comprising as an active ingredient the humanized anti-CD20 monoclonal antibody according to any one of the above-described (1) to (6).

EFFECTS OF THE INVENTION

The present invention provides a monoclonal antibody, in particular, humanized monoclonal antibody, and a method for producing the same, which displays a high binding affinity to an extracellular epitope of a CD20 antigen and possess biological activities, such as inhibitory activity of cell growth, and which is suitable as pharmaceutical use. The present invention also provides a therapeutic agent for the treatment of diseases involving B-cells, comprising such a monoclonal antibody.

BRIEF DESCRIPTION OF THE DRAWINGS

Fig. 1 is a restriction map showing the structure of pNOW-Ab, a vector for expressing a recombinant antibody.
Fig. 2 is a restriction map showing the structure of pNOW, a vector for expressing a protein.
Fig. 3a is a graph showing the results of an apoptosis test.
Fig. 3b is a graph showing the results of an apoptosis test.
Fig. 3c is a graph showing the results of an apoptosis test.
Fig. 3d is a graph showing the results of an apoptosis test.
Fig. 4a is a graph showing the relationship between antibody concentration and ADCC (using RAJI cells).
Fig. 4b is a graph showing the relationship between antibody concentration and ADCC (using WIL2 cells).
Fig. 4c is a graph showing the relationship between antibody concentration and ADCC (using SU-DHL4 cells).
Fig. 4d is a graph showing the relationship between antibody concentration and ADCC (using RC-K8 cells).
Fig. 5a is a graph showing the relationship between E:T ratio and ADCC (using RAJI cells).
Fig. 5b is a graph showing the relationship between E:T ratio and ADCC (using WIL2 cells).
Fig. 5c is a graph showing the relationship between E:T ratio and ADCC (using SU-DHL4 cells).
Fig. 5d is a graph showing the relationship between E:T ratio and ADCC (using RC-K8 cells).
Fig. 6a is a graph showing the results of a CDC test (using RAJI cells).
Fig. 6b is a graph showing the results of a CDC test (using WIL2 cells).

Fig. 6c is a graph showing the results of a CDC test (using SU-DHL4 cells).

Fig. 6d is a graph showing the results of a CDC test (using RC-K8 cells).

Fig. 7a is a graph showing the results of apoptosis experiments using a mouse antibody. The numbers on the right side in the figure represent the concentration of the antibody ($\mu$g/ml), and + and - represent the presence and the absence of crosslinking with a goat antibody, respectively (these representations apply in Fig. 7a to Fig. 7d). The cell used is RAJI cell.

Fig. 7b is a graph showing the results of apoptosis experiments using a mouse antibody. The cell used is WIL2 cell.

Fig. 7c is a graph showing the results of apoptosis experiments using a mouse antibody. The cell used is SU-DHL4 cell.

Fig. 7d is a graph showing the results of apoptosis experiments using a mouse antibody. The cell used is RC-K8 cell.

Fig. 8a is a graph showing the results of apoptosis experiments using a humanized antibody. The numbers on the right side in the figure represent the concentration of the antibody ($\mu$g/ml), and + and - represent the presence and the absence of crosslinking with a goat antibody, respectively (these representations apply in Figs. 8a to 8d). The cell used is RAJI cell.

Fig. 8b is a graph showing the results of apoptosis experiments using a humanized antibody. The cell used is WIL2 cell.

Fig. 8c is a graph showing the results of apoptosis experiments using a humanized antibody. The cell used is SU-DHL4 cell.

Fig. 8d is a graph showing the results of apoptosis experiments using a humanized antibody. The cell used is RC-K8 cell.

Fig. 9a is a graph showing the ratio of early apoptosis using a humanized antibody. The value in the case of adding no antibody is set to 1. The numbers on the right side in the figure represent the concentration of the antibody ($\mu$g/ml), and + and - represent the presence and the absence of crosslinking with a goat antibody, respectively (these representations apply in Figs. 9a to 9d). The cell used is RAJI cell.

Fig. 9b is a graph showing the ratio of early apoptosis using a humanized antibody. The cell used is WIL2 cell.

Fig. 9c is a graph showing the ratio of early apoptosis using a humanized antibody. The cell used is SU-DHL4 cell.

Fig. 9d is a graph showing the ratio of early apoptosis using a humanized antibody. The cell used is RC-K8 cell.

Fig. 10a is a graph showing CDC activities of humanized antibodies and chimerized antibodies. The cell used is Raji cell.

Fig. 10b is a graph showing CDC activities of humanized antibodies and chimerized antibodies. The cell used is SU-DHL4 cell.

Fig. 10c is a graph showing CDC activities of humanized antibodies and chimerized antibodies. The cell used is WiL2 cell.

Fig. 10d is a graph showing CDC activities of humanized antibodies and chimerized antibodies. The cell used is RC-K8 cell.

Fig. 11a is a graph showing the relationship between humanized antibody concentration and ADCC. The cell used is RAJI cell.

Fig. 11b is a graph showing the relationship between humanized antibody concentration and ADCC. The cell used is SU-DHL4 cell.

Fig. 11c is a graph showing the relationship between humanized antibody concentration and ADCC. The cell used is WiL2 cell.

Fig. 11d is a graph showing the relationship between humanized antibody concentration and ADCC. The cell used is RC-K8 cell.

Fig. 12a is a graph showing the results of apoptosis experiments using a humanized antibody. (XL) represents the presence of crosslinking with a secondary antibody (goat anti-human antibody). The cell used is RC-K8 cell.

Fig. 12b is a graph showing the results of apoptosis experiments using a humanized antibody. (XL) represents the presence of crosslinking with a secondary antibody (goat anti-human antibody). The cell used is SU-DHL4 cell.

Fig. 12c is a graph showing the results of apoptosis experiments using a humanized antibody. (XL) represents the presence of crosslinking with a secondary antibody (goat anti-human antibody). The cell used is RAJI cell.

Fig. 12d is a graph showing the results of apoptosis experiments using a humanized antibody. (XL) represents the presence of crosslinking with a secondary antibody (goat anti-human antibody). The cell used is WIL2NS cell.

Fig. 13a is a graph showing the results of apoptosis experiments using a humanized antibody. (XL) represents the presence of crosslinking with a secondary antibody (goat anti-human antibody). The cell used is RC-K8 cell. The activity of inducing apoptosis with no added antibody is set to 1.

Fig. 13b is a graph showing the results of apoptosis experiments using a humanized antibody. (XL) represents the presence of crosslinking with a secondary antibody (goat anti-human antibody). The cell used is SU-DHL4 cell. The activity of inducing apoptosis with no added antibody is set to 1.

Fig. 13c is a graph showing the results of apoptosis experiments using a humanized antibody. (XL) represents the presence of crosslinking with a secondary antibody (goat anti-human antibody). The cell used is RAJI cell. The activity of inducing apoptosis with no added antibody is set to 1.

Fig. 13d is a graph showing the results of apoptosis experiments using a humanized antibody. (XL) represents the presence of cross linking with a secondary antibody (goat anti-human antibody). The cell used is WIL2NS cell. The activity of inducing apoptosis with no added antibody is set to 1.

Fig. 14a is a graph showing CDC activities of humanized antibodies. The cell used is RC-K8 cell.

Fig. 14b is a graph showing CDC activities of humanized antibodies. The cell used is SU-DHL4 cell.

Fig. 15a is a graph showing ADCC activities of humanized antibodies. The cell used is RC-K8 cell.

Fig. 15b is a graph showing ADCC activities of humanized antibodies. The cell used is SU-DHL4 cell.

Description of Abbreviations:

Pcmv: Cytomegalovirus promoter

PAbgh: Poly A addition signal of bovine growth hormone gene

Psvd: Enhancer-deleted simian virus 40 promoter

DHFR: cDNA for mouse dihydrofolate reductase

PAsv: Poly A addition signal from simian virus 40

PBR322ori: E. coli origin of replication

$Amp^r$ : E. coli selective marker (ampicillin resistance)

$Neo^r$ : Mammalian cell selective marker (G418 resistance)

INrbg: Rabbit $\beta$-globin intron

SP1: Antibody light-chain signal peptide

VL: cDNA for a light-chain variable region of an antibody

C$\kappa$: cDNA for a $\kappa$ light-chain constant region of an antibody

SPh: Antibody light-chain signal peptide

Vh: cDNA for a light-chain variable region of an antibody

C$\gamma$1: cDNA for a $\gamma$1 heavy-chain constant region of an antibody

BEST MODE FOR CARRYING OUT THE INVENTION

In this specification, an "antibody" includes not only a whole antibody, but also fragments thereof displaying binding affinities to its antigen which are comparable to that of the whole antibody, such as fragments containing the variable region of the original whole antibody (for example, Fab, F(ab')$_2$, and the like).

Monoclonal antibodies according to the present invention are monoclonal antibodies which display high affinities to human CD20 antigen and have excellent biological activities, and include mouse-derived monoclonal antibodies, and chimerized and humanized variants thereof.

According to a preferred, first embodiment of the present invention, there is provided a monoclonal antibody having a growth inhibiting activity against cells expressing human CD20 antigen and a high affinity to human CD20 antigen, with a dissociation constant (Kd value) for Raji cells (floating cells) of not more than one half of the Kd value of the mouse antibody 2B8, from which Rituximab is derived, preferably with a Kd value between 1.7 and 3.39 nM.

There is no particular limitation on methods for measuring the dissociation constant (Kd value), as long as these methods allow the Kd value to be measured with respect to floating cells. In this specification, however, the dissociation constant is taken to be one obtained by the method described hereinafter in the Examples.

It is preferable that growth inhibiting activity of the antibody of the present invention against cells expressing human CD20 antigen is greater than that of 2B8. The growth inhibiting activity preferably is a growth inhibiting activity with respect to in vitro cultures of cells expressing human CD20 antigen, in the absence of peripheral blood monocytes, more preferably, a growth inhibiting activity due to apoptosis induction.

Measurements of the growth inhibiting activity as described above can be made, for example, using the method described in Miyamoto T, Min W, Lillehoj HS. Avian Dis., 2002 Jan-Mar; 46(1) :10-6.

Specific examples of monoclonal antibodies according to the first embodiment of the present invention include a mouse-derived monoclonal antibody wherein the amino acid sequence of the L-chain variable region and the amino acid sequence of the H-chain variable region are set forth in SEQ ID Nos: 1 and 9, SEQ ID Nos: 2 and 10, or SEQ ID Nos: 3 and 11, respectively, and chimerized or humanized variants thereof.

Chimerization can be carried out, for example, by fusing an amino acid sequence from the variable region of a mouse-derived monoclonal antibody with an amino acid sequence from the constant region of human immunoglobulin, according to known methods as described in Ishida T, Imai K, Nippon Rinsho, Vol. 60, No. 3, 2002-3:439-444.

Humanization can be carried out, for example, by using the CDR amino acid sequence of the variable region of a mouse-derived monoclonal antibody and the amino acid sequence of human immunoglobulin, according to known methods as described in Ishida T, Imai K, Nippon Rinsho, Vol. 60, No. 3, 2002-3:439-444, and also in Eduardo A. Padlan, Molecular Immunology, Vol. 28-4/5, pp. 489-498, 1991; Eduardo A. Padlan et. al., The FASEB Journal, vol. 9, pp. 133-139; and Tai te Wu, Elvin A. Kabat, Molecular Immunology, Vol. 29-9, pp. 1141-1146, 1992.

When chimerization or humanization is carried out, one may combine, in any combination, an amino acid sequence from the L-chain variable region and an amino acid sequence from the H-chain variable region of a plurality of mouse monoclonal antibodies. Examples include, for example, chimerized anti-CD20 monoclonal antibodies combining the L-chain which has a chimerized amino acid sequence from the amino acid sequence of the variable region of a mouse-derived monoclonal antibody set forth in any one of SEQ ID Nos: 1 to 3 and the H-chain which has a chimerized amino acid sequence from the amino acid sequence of the variable region of a mouse-derived monoclonal antibody set forth in SEQ ID Nos: 9 to 11; and humanized anti-CD20 monoclonal antibodies combining the L-chain which has a humanized amino acid sequence of the CDR amino acid sequence of the variable region of a mouse-derived monoclonal antibody set forth in any one of SEQ ID Nos: 1 to 3 and the H-chain which has a humanized amino acid sequence of the CDR the amino acid sequence of the variable region of a mouse-derived monoclonal antibody set forth in any one of SEQ ID Nos: 9 to 11.

An antibody according to a preferred, second embodiment of the present invention is a mouse-derived chimerized or humanized monoclonal antibody which has a dissociation constant (Kd value) for Raji cells, which are floating cells, of not more than one eighth of the Kd value of 2B8.

It is well known that when antibodies having high affinity for human CD20 antigen and containing, in particular, human IgG1 or IgG3 sequence, or an human Fc sequence modified therefrom (including chimerized or humanized versions of mouse-derived monoclonal antibodies) bind to CD20 on the cell surface, they induce activation of effector cells via FcγRIII (CD16) on NK cells, resulting in antibody-dependent cell-mediated cytotoxicity (ADCC). It is well known that when antibodies having high affinity for humanized CD20 antigen and containing, in particular, human IgG or IgM sequence, or a human Fc sequence modified form (including chimerized or humanized versions of mouse-derived monoclonal antibodies) bind to CD20 on the cell surface, they induce activation of complements, resulting in complement-dependent cytotoxicity (CDC).

Therefore, antibodies according to the second embodiment of the present invention can be expected to have a low or undetected growth-inhibiting activity and to display ADCC or CDC.

Specific examples of antibodies according to the second embodiment of the present invention include antibodies wherein the amino acid sequence of the L-chain variable region and the amino acid sequence of the H-chain variable region are set forth in SEQ ID Nos: 4 and 12, SEQ ID Nos: 5 and 13, SEQ ID Nos: 6 and 14, SEQ ID Nos: 7 and 15, or SEQ ID Nos: 8 and 16, respectively.

Chimerization or humanization can be carried out by similar methods as in the antibodies according to the first embodiment. One may combine, in may combination, an amino acid sequence from the L-chain variable region and an amino acid sequence from the H-chain variable region of a plurality of mouse monoclonal antibodies. Examples include, for example, chimerized anti-CD20 monoclonal antibodies combining the L-chain which has a chimerized amino acid sequence from the amino acid sequence of the variable region of a mouse-derived monoclonal antibody set forth in any one of SEQ ID Nos: 4 to 8 and the H-chain which has a chimerized amino acid sequence from the amino acid sequence of the variable region of a mouse-derived monoclonal antibody set forth in SEQ ID Nos: 12 to 16; and humanized anti-CD20 monoclonal antibodies combining the L-chain which has a humanized amino acid sequence of the CDR amino acid sequence of the variable region of a mouse-derived monoclonal antibody set forth in any one of SEQ ID Nos: 4 to 8 and the H-chain which has a humanized amino acid sequence of the CDR amino acid sequence of the variable region of a mouse-derived monoclonal antibody set forth in any one of SEQ ID Nos: 12 to 16.

An antibody according to a preferred, third embodiment of the present invention belongs to a group of humanized monoclonal antibodies which are not limited by a specific dissociation constant (Kd value) for 2B8, including humanized monoclonal antibodies which are effective against cells on which rituximab has no effect.

Examples of such antibodies include humanized anti-CD20 monoclonal antibodies combining the L-chain set forth in SEQ ID No: 18 and the H-chain set forth in SEQ ID No: 24, the L-chain set forth in SEQ ID No: 18 and the H-chain set forth in SEQ ID No: 22, the L-chain set forth in SEQ ID No: 19 and the H-chain set forth in SEQ ID No: 22, and the L-chain set forth in SEQ ID No: 19 and the H-chain set forth in SEQ ID No: 23.

Mouse-derived monoclonal antibodies according to the present invention or mouse-derived monoclonal antibodies which can be used in the preparation of antibodies according to the present invention can be prepared, for example, by selecting a clone producing a monoclonal antibody having desired properties from hybridoma clones obtained by screening with the methods described below.

As sensitizing antigen (immunogen) are used, for example, SB or Raji cells which are CD20 expressing cells and CHO cells which have been transformed by recombinant techniques, for example, using a commercially available DNA for CD20 (or fragments thereof having the same effect) (CHO/CD20),so as to express CD20 on the cell surface. The initial immunization, booster immunization(s), and the final immunization are administered such that the initial immunization and the booster immunization(s) are either carried out at least once using a cell strain which presents the sensitizing antigen and is derived from an animal belonging to a different order from the subject animal for immunization or carried out at least once using a cell strain which presents the sensitizing antigen on the surface of the cell membrane by genetic recombination and is derived from an animal belonging to the same order as the subject animal for immunization, and the final immunization is carried out using the other cell strain.

Other conditions may be similar to conditions used in methods for generating hybridomas producing usual monoclonal antibodies. Hybridomas producing monoclonal antibodies are generated by (1) immunization of animals to be immunized (2) preparation of lymphocytes from the immunized animals, (3) preparation of parent cells, (4) cell fusion of the lymphocytes and the parent cells, and (5) screening and cloning, according to known methods (see, for example, Monokuronaru kotai, Seikagaku Jikkenho [Monoclonal Antibody, Biochemical Experiment Methods], Ailsa M. Campbell (ed.), Toshiaki OSAWA (trans.), Tokyo Kagaku Dojin (1989)).

Methods for preparing monoclonal antibodies using cloned hybridomas can be similar to conventional methods for preparing monoclonal antibodies, except for employing hybridomas prepared using the method of hybridoma production according to the present invention. Large-scale production can be effected, for example, using methods by which the antibody is produced by cell culturing or as mouse ascites. Production of chimerized or humanized antibodies can be performed by preparing a gene coding for the chimerized or humanized antibody, inserting the gene into an expression vector, and expressing the expression vector in a suitable cell.

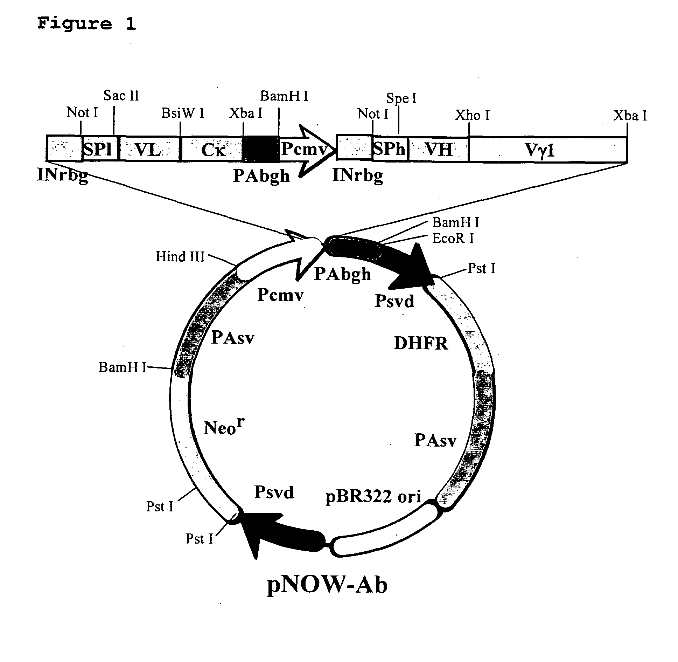

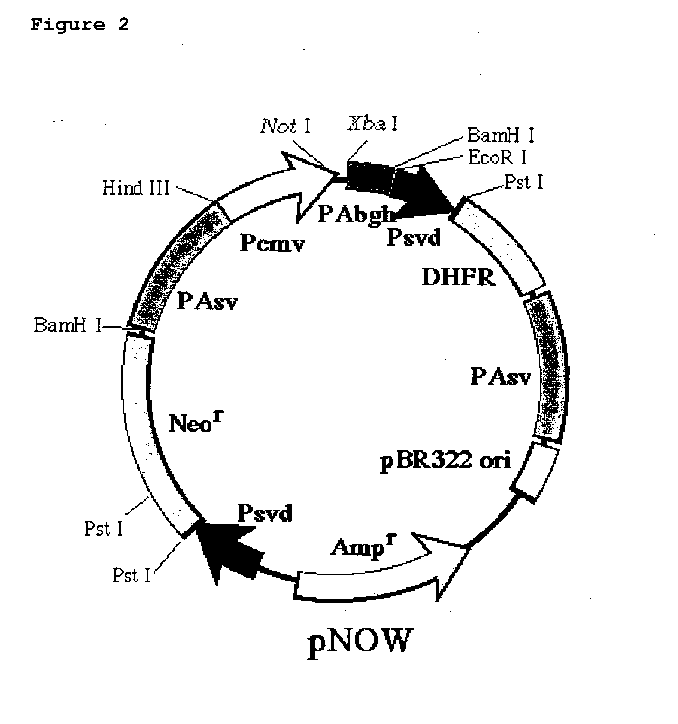

For example, genes for the L-chain variable region and the H-chain variable region can be chimerized using genes for the L-chain constant region and the H-chain ($\kappa$) constant region of human immunoglobulin and inserted into a high expression vector for CHO cell. Although commercially available vector systems for the production of recombinant antibodies may be employed, it is possible to use vectors constructed in pNOW-ab, a dimer high-expression vector containing multicloning sites (MCSs) for both the L-chain and the H-chain, which is based on pNOW, a high expression vector for mammalian cells (Japanese Patent No. 3,582,965). Restriction maps showing the structure of these vectors are shown in Fig. 1 and Fig. 2. CHO cells are transfected with an expression vector containing the chimerized antibody gene, and then highly-productive clones are selected. Antibodies are produced from these clones using usual methods.

Antibodies according to the first embodiment of the present invention display relative high binding affinities, as compared to rituximab, and high growth-inhibiting activities, preferably, due to apoptosis induction, and thus chimerized or humanized antibodies of these antibodies can be used as an active ingredient of therapeutic agents for malignant tumors of B-cells and diseases involving B-cells. Furthermore, antibodies according to the second and third embodiments of the present invention are thought to display complement-dependent cytotoxicity (CDC) or antibody-dependent cell-mediated cytotoxicity (ADCC) against cells expressing human CD20 antigen, and thus can be used as an active ingredient of therapeutic agents for malignant tumors of B-cells and immune diseases involving B-cells. Therefore, the present invention also provides therapeutic agents for diseases involving B-cells which have as an active ingredient the chimerized or humanized antibodies of the invention.

In addition, among the antibodies according to the present invention are antibodies that do not need an antibody requiring crosslinking with a secondary antibody in the induction of apoptosis, and these antibodies are suitable for pharmaceutical use, since antibodies capable of inducing apoptosis by themselves do not require any secondary antibody.

Furthermore, in the present invention, two or more types of antibodies according to the present invention can be combined. Diseases involving B-cells include, but are not limited to, for example, non-Hodgkin's lymphoma, chronic lymphocytic leukemia, acute lymphocytic leukemia, rheumatoid arthritis, autoimmune hemolyticanemia, idiopathic thrombocytopenic purpura, systemic lupus erythematosus, anti-phospholipid antibody syndrome, Sjogren syndrome, Crohn's disease, scleroderma, multiple sclerosis, and others.

The therapeutic agents can be produced by techniques known for pharmaceutical preparations. There is not particular limitation on other formulating ingredients. Dosages and the like can be determined by reference to known Rituxan.

In further embodiments, the present invention provides a humanized anti-CD20 monoclonal antibody combining the L-chain set forth in any one of SEQ ID Nos: 27 to 30 and the H-chain set forth in any one of SEQ ID Nos: 31 to 34 (referred to herein as antibody 1782, antibody of the series 1782, or the like). Of these antibodies, exemplified as preferable antibodies are humanized anti-CD20 monoclonal antibodies in which the L-chain set forth in SEQ ID No: 30 and the H-chain set forth in SEQ ID No: 34 are combined (clone ff); the L-chain set forth in SEQ ID No: 30 and the H-chain set forth in SEQ ID No: 31 are combined (clone fv); and the L-chain set forth in SEQ ID No: 29 and the H-chain set forth in SEQ ID No: 33 are combined (clone ss). These humanized antibodies, which are also derived from mouse, can be humanized as described above.

The series 1782 of humanized antibodies has weak activity for inducing apoptosis by themselves, but display, under crosslinking conditions, apoptosis inducing activity equal to or higher than that of Rituxan (c2B8). Humanized antibody 1782 displays CDC activity not only against RAJI, WIL2NS, and SU-DHL4 cells, but also against RC-K8 cells which are of a Rituxan-resistant strain. In particular, as detailed in the Examples, clones ff and fv of the series 1782 have high CDC activities and display significantly greater CDC activities against SU-DHL4 cells than Rituxan. Putting together the results of Kd values and CDC activities, the clone ff of the series 1782 is preferable in that it has the highest affinity, a relatively high CDC activity, and a CDC activity also against the strain RC-K8, a Rituxan-resistant strain. The clone fv of the series 1782 is also preferable in that it has the highest CDC activity and in addition, is effective also on the strain RC-K8, a Rituxan-resistant strain. In particular, since the clone ff of the series 1782 has a very high affinity, it is possible to RI label it to employ it for missile therapy of B-cell involving diseases. Furthermore, the clone fv of the series 1782 is preferable in that it has a high CDC activity against SU-DHL4 cells, and the clone ss is preferable in that it has a high ADCC activity against RC-K8 cells.

The series 1782 of humanized anti-CD20 monoclonal antibodies according to the present invention can be also used as an active ingredient of therapeutic agents for the treatment of malignant tumors of B-cells and immune diseases involving B-cells. Therefore, the present invention, in further embodiments, provides therapeutic agents for the treatment of malignant tumors of B-cells and immune diseases involving B-cells, in which the therapeutic agents contain an antibody of the series 1782 as an active ingredient. Methods by and amounts at which the therapeutic agents are administered can be easily determined by those skilled in the art. As diseases involving B-cells are exemplified the above-described diseases.

The present invention will be described in more detail hereinafter with reference to the Examples. However, the invention is not limited to the Examples.

EXAMPLE 1

(1) Preparation of Immunization Agents for Sensitizing Mice

SB and Raji cells, which are of B-cell strains expressing CD20, were cultured *in vitro.*
Separately, DNA coding for the entire CD20 molecule (Multiple Choice cDNA human spleen, Origene Technologies, Inc., 6 Taft Court, Suite 100, Rockville, MD 20850) was cloned using specific primers hCD20-S-GK-Not: aatgcggccgccaccatgacaacacccagaaattc (SEQ ID No: 25) and hCD20-E-Xba: gctctagattaaggagagctgtcattttc (SEQ ID No: 26). The DNA was inserted into a high expression vector for mammalian cells, pNOW, (Fig. 1) and a constructed vector was used to transform CHO cells. Recombinant CHO cells (CD20/CHO cells) displaying high levels of expression of CD20 on the cell surface were identified using FACS analysis. Staining was performed using an FITC-labeled anti-CD20 monoclonal antibody and cells were selected as high expression cell, when they gave five or more times the fluorescence intensity of SB cells.

(2) Preparation of Immunogens

SB or Raji cells were cultured using RPMI 1640 medium supplemented with 10% FCS. CD20/CHO cells were cultured using CHO-S-SFM II medium (Gibco, Cat. No. 12052-098) supplemented with 800 $\mu$g/ml of G418. These cultured mediums were centrifuged for 5 minutes at 1,100 rpm, the cells were suspended in Dulbecco's PBS(-) and centrifuged again. This washing step was repeated once again, and physiological saline was added to the cells to prepare a suspension (cell number: 1-3 x $10^7$/ml), which was used for immunization.

(3) Immunization

These immunogen preparations were administered intraperitoneally to female Balb/c mice of 7- to 11-week old. After administration of either of the SB or CD20/CHO cells was repeated two to three times at intervals of various days, the final immunization was carried out using cells of a different type (CD20/CHO or Raji cells). The number of cells administered was 1-3 x $10^7$ cells per mouse for each of these immunizations.
The combinations of immunogens used are shown in Table 1.

(4) Cell Fusion

Three days after the final immunization, spleen cells were prepared from two mice and fused with mouse myeloma cells (NS-1) in the presence of PEG-1500 according to the method described in Oi, V.T. and L.A. Herzenberg, 1980, in: Selected Methods in Cellular Immunology, eds.: B. Mishell and S.M. Shiigi (Freeman and Co., San Francisco, CA) p.351.

(5) Primary and Secondary Screening

Cell ELISA was performed using 96-well plates having CD20/CHO or CHO cells (parent strain) attached thereto to select wells where an antibody reacting specifically to CD20 was produced. 96-well plates with the same CD20/CHO cells attached thereto were subjected competitive reaction with rituximab (C2B8) to select antibodies (wells) which reacted to an epitope similar to that of C2B8.

The results of these screenings are shown in Table 1.

(6) Cell ELISA

CD20/CHO or CHO cells (parent strain) attached to a Poly-L-Lysine coated 96-well plate (Asahi Technoglass Corporation, Cat. No. 11-023-018) were used for Cell ELISA. 150 μl of blocking buffer (PBS solution with 0.2% gelatin, 0.5% BSA) was placed into each well and the plate was allowed to stand at 37°C for one hour. The plate was washed five times using an aqueous solution of 150 nM NaCl, 0.05% Tween 20, and then a 100-μl sample (a diluted solution of a cultured supernatant) was placed into each well. The primary reaction was conducted at 37°C for one hour. After washing, 100 μl of a diluted solution of a labeled antibody (HRP-labeled anti-mouse IgG (H+L) rabbit antibody (Jackson Lab., Code No. 315-035-003) or HRP-labeled anti-mouse IgG (Fcγ) rabbit antibody (Jackson Lab., Code No. 315-035-008)) was placed into each well and a secondary reaction was conducted at 37°C for one hour. The same blocking solution was used in the preparation of the reaction solution for the primary and the secondary reactions. After washing, 100 μl of a color developing solution (OPD) was placed into each well and 30 minutes later, 50 μl of 4N $H_2SO_4$ was added to stop the reaction.
The absorbance was measured at 492 nm.

(7) Competitive Reaction in Cell ELISA

Mixed solutions of a sample (diluted solution of a cultured supernatant) and a chimerized antibody (10 to 40 ng/ml) were prepared.
After blocking reaction was carried out as in the above-described Cell ELISA, 100 μl of each of the mixed solutions was placed into each well and the primary reaction was performed at 37°C for one hour. After washing, 100 μl of a diluted solution of a labeled antibody (HRP-labeled anti-human IgG (H+L) rabbit antibody (Jackson Lab., Code No. 309-035-082)) was placed into each well and a secondary reaction was conducted at 37°C for one hour. After washing, 100 μl of a color developing solution (OPD) was placed into each well and 30 minutes later, 50 μl of 4N $H_2SO_4$ was added to stop the reaction. The absorbance was measured at 492 nm.
Since the labeled antibody reacts only with the chimerized antibody, any competition between the antibody in the sample added in the primary reaction and the chimerized antibody results in reduction in the measured value.

(8) Cloning

A limiting dilution method was used. After culturing cells seeded on 96-well plates, Cell ELISA was performed on cultured supernatants of wells having a single colony, thereby to select clones producing a specific antibody.

(9) Preparation of Purified Antibodies

Clones producing a specific antibody were cultured in RPMI 1640 medium supplemented with 10% FCS. When the cell density reached approximately $5 \times 10^5$ cells/ml, the medium was replaced by serum-free culture medium ASF-104N (Ajinomoto). Two to four days later, the cultured medium was centrifuged to collect the cultured supernatant. A protein G column was used for purification of the antibody. A solution of the eluted monoclonal antibody was dialyzed using 150 mM NaCl. The dialyzed solution was filter sterilized through a 0.2 μm filter to obtain an antibody sample to be tested (anti-human CD20 mouse monoclonal antibody).
Table 1

| Cell Fusion Series | Immunizing Method | | | Primary, Secondary Screening Specificity against CD20 on CD20/CHO cells | | |
|---|---|---|---|---|---|---|
| | Initial and Booster Immunization, Number of Immunizations | Final Immunization | Number of immunized mice | Number of selected wells | | Number of measured wells |
| | | | | A | B | |
| 1K18 | SB cell, 3 times | Raji cell | 2 | 7 | 2 | 576 |
| 1K20 | Raji cell, 3 times | SB cell | 2 | 7 | 0 | 576 |

(continued)

| Cell Fusion Series | Immunizing Method | | | Primary, Secondary Screening Specificity against CD20 on CD20/CHO cells | | |
| --- | --- | --- | --- | --- | --- | --- |
| | Initial and Booster Immunization, Number of Immunizations | Final Immunization | Number of immunized mice | Number of selected wells | | Number of measured wells |
| | | | | A | B | |
| 1K14 | SB cell, 2 times | CD20/CHO cell | 1 | 20 | 9 | 576 |
| | SB cell, 3 times | CD20/CHO cell | 1 | | | |
| 1K17 | CD20/CHO cell 2 times | Raji cell | 1 | 21 | >10 | 576 |
| | CD20/CHO cell 3 times | Raji cell | 1 | | | |

Number of selected wells-A: wells where an antibody reacting with CD20/CHO cells and not with CHO cells was produce.
Number of selected wells-B: of the wells selected in A, wells where an antibody undergoing a competitive reaction with the reference antibody (C2B8) was produce.
The amino acid sequences of the L-chain V-region (SEQ ID Nos: 1 to 8) and the H-chain V-region (SEQ ID Nos: 9 to 16) of monoclonal antibodies produced by 8 representative clones are shown below.

The sequence of the H-chain V-region of 1K0924 (SEQ ID No: 11) :

QVQLQQPGAELVKPGASVKMSCKASGYTFTSYNIHWVKQTPGQGLEWIGAIYPGNGDTS
YNQKFKGKATLTSDKSSSTAYMQLSSLTSEDSAVYYCARMSTMITGFDYWGQGTTLTVS S

The sequence of the H-chain V-region of 1K1228 (SEQ ID No: 16):

QVQLQQPGAELVKPGASVKVSCKASGFTFTSYNLHWVKQTPGQGLVWIGAIYPGNGDTS
YNQKFRGKATLTADISSSTAYMQLSSLTSEDSAVYYCARYYYGYDAMDYWGQGTSVTVS S

The sequence of the H-chain V-region of 1K1422 (SEQ ID No: 9):

QVQLQQPGAELVKPGASVKMSCRASGYTFTNYNMHWIKQTPGQGLEWIGAIYPGSGDTS
YNRKFKGKATLTADTSSSTAYMQFSSLTSADSAVYYCARFTYYYGGTYGAMDYWGQGTS VTVSL

The sequence of the H-chain V-region of 1K1791 (SEQ ID No: 10):

QIQLVQSGPELKKPGETVKISCKASGYTFTNFGVNWVKQAPGKGLKWMGWINTYTGEPS
YADDFKGRFAFSLEASANTAYLQINNLKNDDMSTYFCTRRTNYYGTSYYYAMDYWGQGT SVTVSS

The sequence of the H-chain V-region of 1K1712 (SEQ ID No: 12):

QVQLQQPGAELVKPGASVKMSCKASGFTFTSYNLHWVKQTPGQGLEWIGAIYPGSGDTS
YNQQFKGKATLTADKSSNTAYMQLNSLTSEDSAVYCCARSAMISTGNWYFDYWGQGTTL TVSS

The sequence of the H-chain V-region of 1K1402 (SEQ ID No: 13): .

QVQLQQPGAELVKPGASVKMSCKASGFTFTSYNMHWVKQTPGQGLEWIGGIYPGNGDTS
YNQKFKGKATLTADKSSSTAYMQLSSLTSEDSAVYYCARFYYYGSMGAMDYWGQGTSVT VSS

The sequence of the H-chain V-region of 1K1736 (SEQ ID No: 14):

QVQLQQPGAELVKPGASVKMSCKASGYTFTTYNLHWVKQTPGQGLEWIGGIYPGNGDTS

YNQKFKVKATLTADKSSNTAYMQLSSLTSEDSAVYYCARWIYYGNYEGTLDYWGQGTSV TVSS

The sequence of the H-chain V-region of 1K1782 (SEQ ID No: 15):

QVQLQQSGAELAKPGASVKMSCKASGYTFTSYWMHWVKQRPGQGLEWIGYITPSTGYTD
YNKKFKDKATLTADRSSSTAYMHLSSLTSEDSAVYYCARSGPYFDVWGAGTTVTVSS

The sequence of the H-chain V-region of 1K0924 (SEQ ID No: 3):

QIVLSQSPAILSASPGEKVTMTCRASSSVSYMHWYQQRPGSSPKPWIYATSNLASGVPA
RFSGSGSGTSYYFTISRVEAEDAATYYCQQWNSNPPTHGGGTKLEIK

The sequence of the H-chain V-region of 1K1228 (SEQ ID No: 8):

EIILTQSPTTMAASPGEKITITCSASSSISSYYLRWYQQKPGFSPKVLIYRTSNLASGV
PARFSGSGSGTSYSLTIGTMEAEDVATYYCQQGNTVPLTFGSGTKLEIK

The sequence of the L-chain V-region of 1K1422 (SEQ ID No: 1):

QIVLTQSPPIMSASLGEEITLTCSASSRVSYMLWYQQKSGTSPKLLIYSTSNLASGVPS
RFSGSGSGTFYSLTISSVEAEDAADYYCHQWTSNPCTFGGGTKLEIK

The sequence of the L-chain V-region of 1K1791 (SEQ ID No: 2):

STVMTQTPKFLLVSAGDRVTITCKASQSVSNDVAWYQQKPGQSPKVLIYFASNRYTGVP
DRFTGSGYGTDFTFTINTVQAEDLAVYFCQQDYSSPLTFGAGTKLELK

The sequence of the L-chain V-region of 1K1712 (SEQ ID No: 4):

QIVLSQSPAILSASPGEKVTMTCRASSSVSYMDWYQQKPGSSPKPWIYATSNLASGVPA
RFSGSGSGTSYSLTISRVEAEDTATYYCQQWTFNPPTFGSGTKLEIK

The sequence of the L-chain V-region of 1K1402 (SEQ ID No: 5):

QIVLSQSPAILSASPGEKVTMTCRASSSVSYMHWYQQKPGSSPKPWIYATSNLASGVPA
RFSGSGSGTSYSLTITRVEAEDAATYYCQQWTFNPPTFGAGTKLELK

The sequence of the L-chain V-region of 1K1736 (SEQ ID No: 6):

QIVLSQSPAILSSSPGEKVTMTCRASSSVSYMLWYQQKPGSSPEPWIYATSNLASGVPA
RFSGGGSGTSYSLTISRVEAEDAATYYCQQWTFNPPTFGGGTKLEIK

The sequence of the L-chain V-region of 1K1782 (SEQ ID No: 7):

DILLTQSPAILFVSPGERVSLSCRASQNIGTSIHWYQQRTNGSPRLLIKYASESFSGIP
SRFSGSGSGTDFTLSINSVESEDIADYYCQQSNSWPFTFGSGTKLEIK

EXAMPLE 2

For some of the resulting clones, the base sequences of their monoclonal antibody genes in the variable region were determined. In addition, antibody binding affinities were measured and biological property tests were performed for the monoclonals produced by them, as described below.

(1) Measurement of Binding Affinity

Floating Raji cells derived from human B-cell, which express the target antigen on the cell surface, and floating Jurkat cells derived from human T-cell, which do not express CD20 antigen were used. Cells of both types were cultured in a $CO_2$ incubator (SANYO MCO-175M) at 37°C and at a $CO_2$ concentration of 5% using RPMI 1640 medium (NACALAI TESQUE, Inc., Cat. No. 30264-85, Lot L4K2844) supplemented with 10% fetal calf serum (FCS) (BIOLOGICAL IND., Cat. No. 04-001-1A, Lot 815242; preheated at 56°C for 30 minutes for inactivation of the complement components). The cells were maintained by subculturing twice a week.

Measurements of the number of cells were made using a Burker-Turk hemacytometer (Erma, Inc., Cat. No. 03-303-1). Cultured media of confluent cells three to four days after subculturing were centrifuged for three minutes at 3,000 rpm at room temperature using a multi-rack refrigerated centrifuge LX-120 (TOMY Co., Ltd.). The supernatant was removed and the cells were collected. The rotation speed and time used in this step were selected so that the number of cells remained unchanged after the repetition of centrifugal separation and supernatant removing. For removing the culture medium and FCS remaining on the cell surface (washing), the collected cells were suspended in Dulbecco's Phosphate Buffered Saline (-) (free of Ca and Mg, PBS(-), (NaCl: Wako, Cat. No. 191-01665, $Na_2HPO_4$: Wako, Cat. No. 197-02865, Lot ASF2635, KCl: Wako, Cat. No. 163-0334T, Lot CEQ7122, $KH_2PO_4$ : Wako, Cat. No. 169-0425, Lot ELG7616)) and centrifuged twice for 3 minutes at 3,000 rpm to remove the supernatant. The cells after washing were suspended in a solution of 1% BSA (Wako, Cat. No. 013-07492 Lot PKH3483) in PBS and adjusted to a cell density of $5\times10^6$ cells/ml. As a primary antibody, an antibody to be tested or a positive control antibody (2B8) was dispensed into 1.5-ml tubes (BM Equipment Co., Ltd., BM ring-lock tubes, Cat. No. BM-15) in the respective amounts of 15, 30, 50, 75, 100, 125, 150, and 200 ng (1.5 to 5 µl). At the same time, four tubes with no added antibody were prepared. Three samples were prepared for each antibody to be tested. To each of the samples was added 100 µl ($5\times10^5$ cells) of a suspension in a PBS solution of 1% BSA (Wako, Cat. No. 013-07492, Lot PKH3483) and mixed, and reacted with shaking at room temperature for one hour.

After reacting, the reaction solutions were centrifuged at 3,000 rpm for 3 minutes at room temperature using a high-speed refrigerated microcentrifuge MX-100 (TOMY). After collecting the cells, the cells were suspended in 200 µl of PBS and centrifuged at 3,000 rpm for three minutes to remove the supernatant. These procedures were repeated twice to remove any unreacted primary antibody remaining on the cell surface.

An FITC-labeled anti-mouse IgG (H&L) secondary antibody [GOAT Anti-mouse IgG (H&L) Fluorescein conjugated, affinity purified Secondary antibody, Chemicon, Cat. No. AP124F, Lot 24021014] was added in excess (500 ng) with respect to the primary antibody binding to cells, followed by 100 µl of a 1% BSA-PBS solution (500 ng/100 µl). The mixture was shaken for one hour at room temperature while being shielded from the light, and the primary antibody binding to cells was detected. After the reaction, the mixture was centrifuged at 3,000 rpm for three minutes to collect the cells. In order to remove any unreacted FITC-labeled anti mouse IgG (H&L) antibody remaining on the cell surface, the cells were suspended in 200 µl of PBS and centrifuged at 3,000 rpm for three minutes to remove the supernatant. These procedures were repeated twice.

The cells thus collected were suspended in 100 µl of PBS and transferred to flat-bottomed 96-well plates (Sumitomo Bakelite Co., Ltd., ELISA PLATE, Cat. No. 8496F). The intensity of fluorescence from the secondary antibody was measured using a Typhoon- 9210 Image Analyzer (Amersham Bioscience) under the following detection conditions: Fluorescence mode, 600 V, 526SP/green(532 nm), Focus: +3 mm above the bottom face. Controls for constructing a standard curve were prepared using PBS solutions of 100 µl, to which 0, 12.5, 25, 50, 75, 100, 125, and 150 ng of the FITC-labeled secondary antibody were added.

After the detection, images were digitized using an image analysis software, Image Quant (Amersham Bioscience), and analyzed using Excel (Microsoft). Background values resulting from the plate, PBS solution, and FITC-labeled secondary antibody binding non-specifically to cells were determined by measuring reactions only between the cells and the FITC-labeled secondary antibody, and the average value for the four points were subtracted from the value of fluorescence intensity for each sample. In this way, the amount of fluorescence from the FITC-labeled secondary antibody binding to cells was obtained. A standard curve was constructed by measuring amounts of fluorescence at various concentrations of the FITC-labeled secondary antibody used as control, and the amount of secondary antibody binding to cells (number of moles or weight) was calculated. The amount of primary antibody binding to cells was calculated assuming that each of the primary antibodies and the FITC-labeled secondary antibody react at a ratio of 1: 2. The amount of free primary antibody was determined by subtracting the binding amount from the added amount. When the antibody concentration was converted into molar concentration, the molecular weights of the monoclonal antibodies were taken to be 150,000.

It was observed that the binding reaction became saturated with increasing amounts of primary antibody added and the intensity of fluorescence reached a constant value. Scatchard analysis was used to calculate the number of antigens on the cell surface and the dissociation constant (Kd value) (see Scatchard, G., Ann. N.Y. Acad. Sci., 51: 660-672, 1949; New Cultured Cell Experimental Methods in Molecular Biology Research, Yodosha Co., Ltd., Jikken Igaku separate volume, BioManual UP Series Revised 2nd Edition, pages 212 to 217). The values used were the average of three values for the respective samples.

The measurement results for monoclonal antibodies produced by 8 representative clones and a positive control antibody (2B8) are shown below in Table 3.

(2) Biological Property Tests

(a) Apoptosis Induction Test

The ability of test antibodies to induce apoptosis was measured using flow cytometry (Annexin V/PI staining). A positive control (2B8) and a negative control (Anti-CD3 monoclonal antibody (BD PharMingen) were used. The test was performed using a MEBCYTO Apoptosis Kit (MBL, Cat. No. 4700, Lot. 20).

Raji cells were centrifuged, and suspended in fresh RPMI 1640 medium (Sigma, Cat. No. R8758, Lot. 44K2416) supplemented with 10% (inactivated) FBS (ICN, Cat. No. 2916754, Lot. 8005C) and placed into each well of 12-well plates in a volume of 1 ml at a density of $5 \times 10^5$ cells/ml. Twelve wells per antibody were used for testing, and an antibody was added to the respective wells to give a final concentration of 2 or 4 $\mu$g/ml (3 wells x 2 concentrations x 2 time points, 12 wells in total). On days 1 and 2 after the culture was started, the cultured media containing about $2 \times 10^5$ cells were removed and after centrifugation, the cells were washed once in PBS. Then, the cells were suspended in 85 $\mu$l of binding buffer. After mixing well with 10 $\mu$l of Annexin V-FITC and 5 $\mu$l of PI, the mixture was allowed to react for 15 minutes at room temperature while being shielded from the light. Measurements were made using flow cytometry (FACS Calibur, Becton Dickinson) and analyzed with CellQuest (Becton Dickinson).

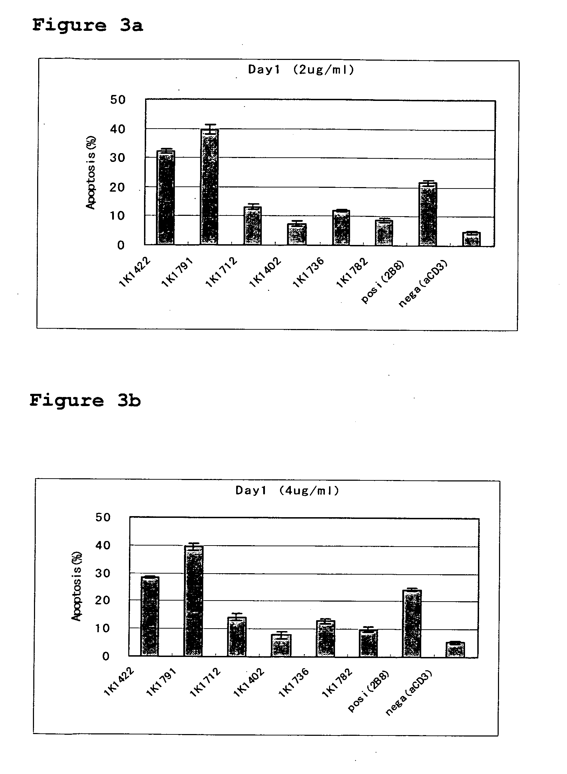

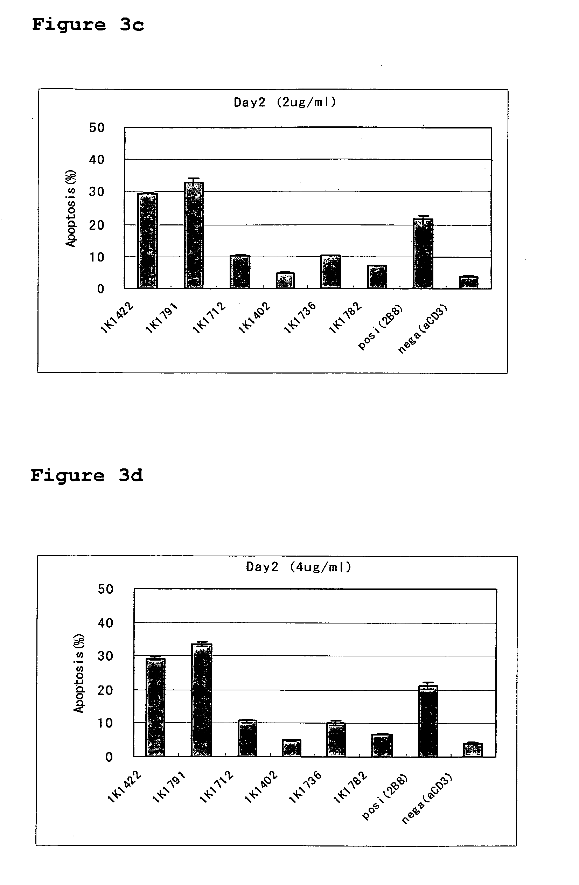

The measurement results for monoclonal antibodies produced by 6 representative clones, a positive control antibody (2B8), and a negative control antibody (Anti-CD3) are shown below in Fig. 3a to Fig. 3d. Although 2B8 is in general believed to have a high ability to induce apoptosis, the monoclonal antibodies produced by the clone 1k1791 obtained from cell fusion of the series 1K17 (immunization with CD20/CHO cells and Raji cells) and by the clone 1k1422 obtained from cell fusion of the series 1K14 (immunization with SB cells and CD20/CHO cells) were found to display high abilities to induce apoptosis when compared with 2B8.

(b) Cell Growth Inhibition Test

A suspension of Raji cells with a cell concentration of $5 \times 10^4$ cells/ml was prepared in RPMI 1640 supplemented with 10% FCS, added at a volume of 100 $\mu$l per well into 96-well plates, and cultured. After 24 hours, an antibody solution was added at a volume of 50 $\mu$l per well to give an antibody concentration of 1 $\mu$l/ml, and culturing was continued. At 72 hours after addition of the antibody, 10 $\mu$l/well of a color developing dye, Cell Counting Kit-8 (Dojindo Laboratories, Cat. No. 343-07623, Lot. SG076), was added, culturing was continued for another 4 hours, and then absorption was measured at 492 nm.

The absorbance measurement results for monoclonal antibodies from the six clones as described above, a positive control antibody (2B8), and a negative control are shown below in Table 2, and their properties are shown in Table 3.

Cell Growth Inhibition Test

Table 2

| Clone name | Absorption (492 nm) |
|---|---|
| 1K1422 | 1.775 |
| 1K1791 | 1.794 |
| 1K1712 | 2.326 |
| 1K1402 | 2.540 |
| 1K1736 | 2.239 |
| 1K1782 | 2.603 |
| Positive control (2B8) | 1.759 |
| Negative control | 2.607 |

Properties of Monoclonal Antibodies

Table 3

| Clone name | Isotype | Binding affinity Kd value (nM) | Ability to Induce Apoptosis | Cell Growth Inhibiting Effect (in Vitro) |
|---|---|---|---|---|
| 1K1422 | IgG1, $\kappa$ | 3.39 | 130 | present |

(continued)

| Clone name | Isotype | Binding affinity Kd value (nM) | Ability to Induce Apoptosis | Cell Growth Inhibiting Effect (in Vitro) |
|---|---|---|---|---|
| 1K1791 | IgG1, κ | 1.70 | 160 | present |
| 1K0924 | IgG2b, κ | 1.35 | 60 | present |
| 1K1712 | IgG2a, κ | 0.84 | 50 | - |
| 1K1402 | IgG1, κ | 0.78 | 30 | - |
| 1K1736 | IgG2b, κ | 0.54 | 50 | - |
| 1K1782 | IgG1, κ | 0.40 | 30 | - |
| 1K1228 | IgG1, κ | 0.26 | 30 | - |
| Positive control (2B8) | IgG1, κ | 6.79 | 100 | present |

(c) Antibody-Dependent Cell-Mediated Cytotoxicity (ADCC) In this series of experiments, measurements were made of whether effector cells could become activated through the Fc region of an anti-CD20 chimerized antibody, thereby to lyse cells of lymphoma cell lines.

Four types of cells, Raji, WiL2-NS, SU-DHL4, and RC-K8 cells derived from human B-cell, were cultured and maintained in an incubator at 37°C and at a $CO_2$ concentration of 5% in RPMI 1640 medium supplemented with 10% inactivated FCS (culture medium).

In these experiments, cells of each type were washed in RPMI 1640 containing 10% FCS and reacted with calcein for 15 minutes at 37°C, whereby the calcein was allowed to be incorporated into viable cells, and after that, the number of cells was adjusted to $4 \times 10^5$ cells/ml. Since calcein is retained only in cells having the cell membrane kept in normal conditions, it can be used to stain only viable cells. Rituximab (C2B8), which is an anti-CD20 chimerized antibody, and 6 types of chimerized antibodies (1k0924, 1k1402, 1k1422, 1k1712, 1k1736, and 1k1791) were adjusted to be at 20, 4, and 0.8 μg/ml using RPMI 1640 containing 10% FCS. Effector cells were adjusted to be at $5 \times 10^6$, $1 \times 10^6$, and $0.2 \times 10^6$ cells/ml after effector cells were obtained by taking blood from healthy subjects and immediately layering the blood onto a Ficoll, followed by centrifugation to collect a lymphocyte fraction.

In a total of 100 μl, 25 μl of a cell suspension having an adjusted concentration, 25 μl of each of the anti-CD20 chimerized antibody solutions (with the respective final concentrations of 5, 1, and 0.2 μg/ml), and 50 μl of effector cell for each of the antibody concentrations (with the respective E:T ratios of 25:1, 5:1 and 1:1) were mixed well in 96-well plates and reacted for 4 hours in an incubator at 37°C and at a $CO_2$ concentration of 5%. Three samples were prepared as follows: a first sample in which the antibody solution and the effector cells were replaced with RPMI 1640 containing 10% FCS, in order to calculate the spontaneous lysis of cells; a second sample in which the antibody solution was replaced with RPMI 1640 containing 10% FCS, which was for calculating the antibody-independent activity of only the effector cells; and a third sample in which the antibody solution was replaced with 20% Triton X-100, which was for calculating the maximum lysis.

After the reaction, if cells have been lyzed, then the cell membrane has been broken and the calcein has been released out of the cell. Based on this, the fluorescence of calcein released into the reaction solution was quenched using a Quencher and the amount of fluorescence was then measured using a fluorescence analyzer.

After the detection, images were digitized using an image analysis software (Amersham Bioscience), and the lysis rate for each sample was calculated using the equation below.

```
Equation 1

Lysis rate (%) = ((Spontaneous lysis) -

(Sample))/((Spontaneous lysis) - (Maximum lysis)) x 100
```

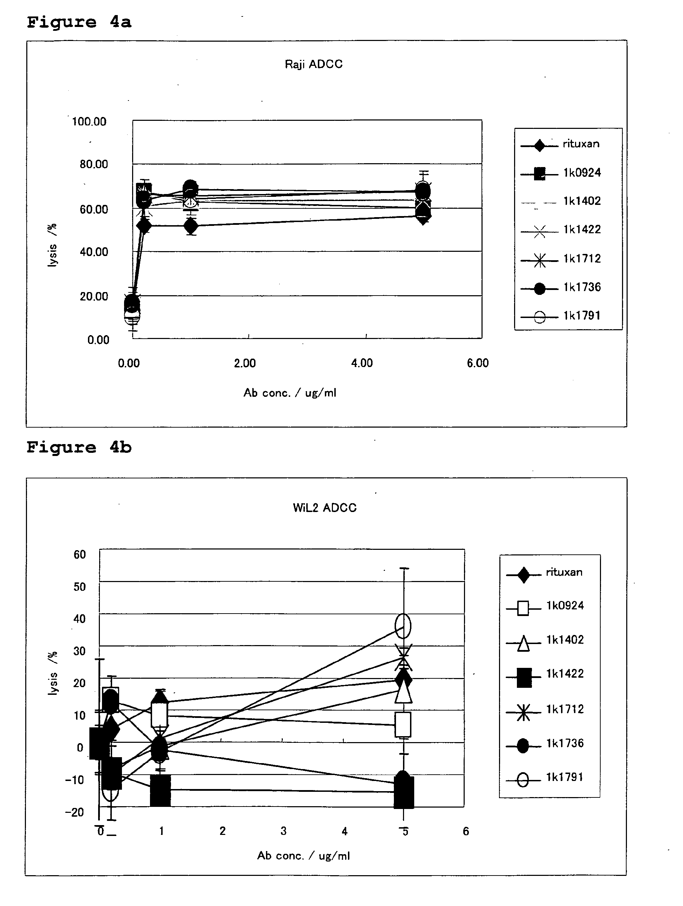

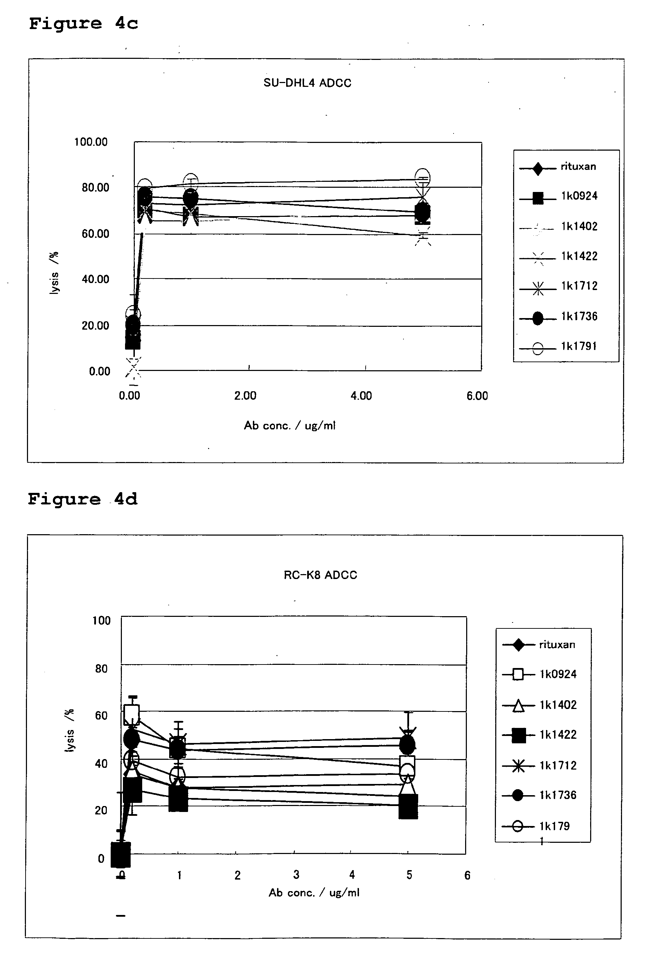

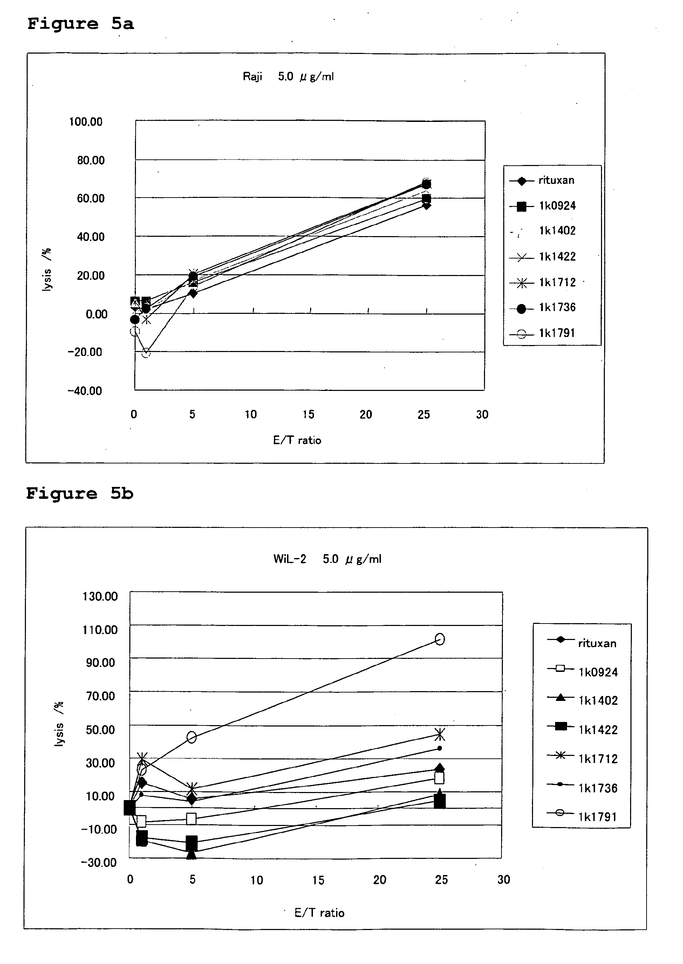

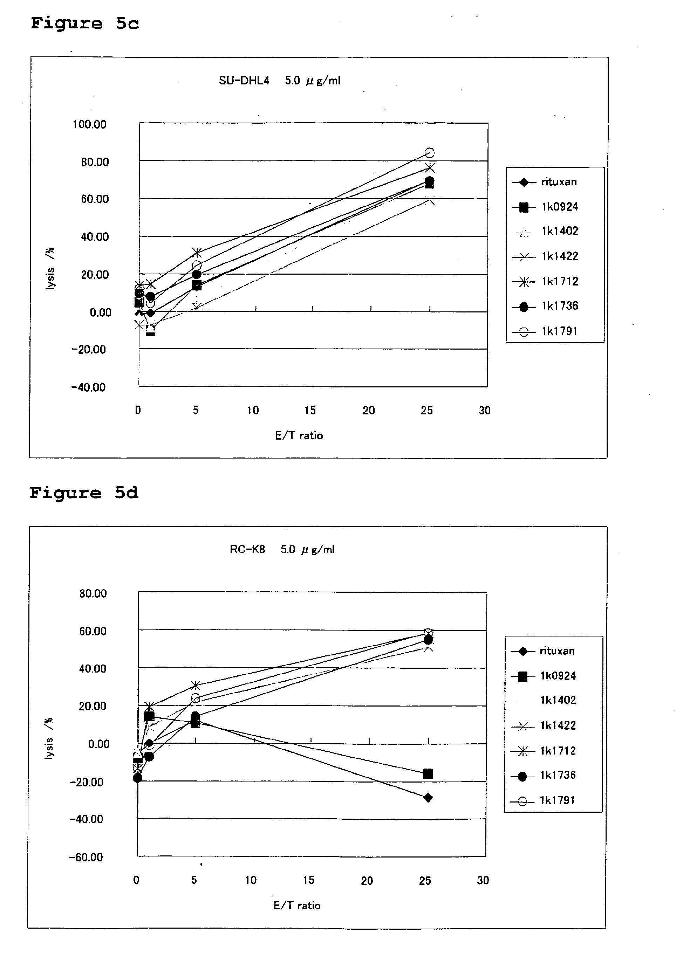

Fig. 4a to Fig. 4d show the relationship between antibody concentration and cytotoxic activity for each of the cell types, when the ratio of the number of effector cells, such as NK cells, and the number of target cells (E:T ratio) was 25:1. Fig. 5a to Fig. 5d show the relationship between the E:T ratio and cytotoxic activity for each of the cell types, when the antibody concentration was 5 μg/ml.

As shown in Fig. 4a to Fig. 4d, all of the cell types shows cytotoxic activity by adding the respective antibodies, under conditions where the E:T ratio was 25:1. In other words, the antibodies participate in cytotoxicity. Furthermore, cell strains other than WiL2-NS display, already at an antibody concentration of 0.2 $\mu$g/ml, activities equivalent to those at antibody concentrations of 1 and 5 $\mu$g/ml (which become saturated at 0.2 $\mu$g/ml) and the activity becomes constant after reaching a maximum, suggesting that these effects are provided in amounts of antibody which are less than the amount of antibody required for complement-dependent cytotoxic activity.

As shown in Fig. 5a to Fig. 5d, it would be recognized that from the effect of E:T ratio on cytotoxic activity at an antibody concentration of 5 $\mu$g/ml, the cytotoxic activity increases depending on the E:T ratio concentration. From this, it can be concluded that the cytotoxicity is caused by the action of effector cells.

(d) Complement Dependent Cytotoxicity (CDC)

In this series of experiments, measurements were made of whether the anti-CD20 chimerized antibodies could lyse cells of lymphoma cell lines in the presence of serum containing complements.

Four types of cells, Raji, WiL2-NS, SU-DHL4, and RC-K8 cells derived from human B-cell, were cultured and maintained in an incubator at 37°C and at a $CO_2$ concentration of 5% in RPMI 1640 supplemented with 10% inactivated FCS (culture medium).

In these experiments, cells of each type were washed in RPMI 1640 containing 10% FCS and the number of cells was adjusted to 2-3 x $10^6$ cells/ml. C2B8 (rituximab), which is an anti-CD20 chimerized antibody, and 6 types of chimerized antibodies (1k0924, 1k1402, 1k1422, 1k1712, 1k1736, and 1k1791) were adjusted to be at 20, 4, and 0.8 $\mu$g/ml using RPMI 1640 containing 10% FCS.

In a total of 100 $\mu$l, 55 $\mu$l of a cell suspension having an adjusted concentration, 25 $\mu$l of each of the anti-CD20 chimerized antibody solutions (with the respective final concentrations of 5, 1, and 0.2 $\mu$g/ml), and 20 $\mu$l of pooled serum taken from five healthy subjects or an inactivated serum thereof were mixed well using a vortex mixer and reacted for 2 hours in an incubator at 37°C and at a $CO_2$ concentration of 5%. Samples in which 25 $\mu$l of the antibody solution was replaced with RPMI 1640 containing 10% FCS were prepared as samples for calculation of backgrounds.

After the reaction, dead cells were stained using PI (propidium iodide) and analyzed by FACS (Becton Dickinson). As numerical values, populations obtained of dead cells were directly used, from which the values of background and of the sample to which the inactivated serum was added were subtracted.

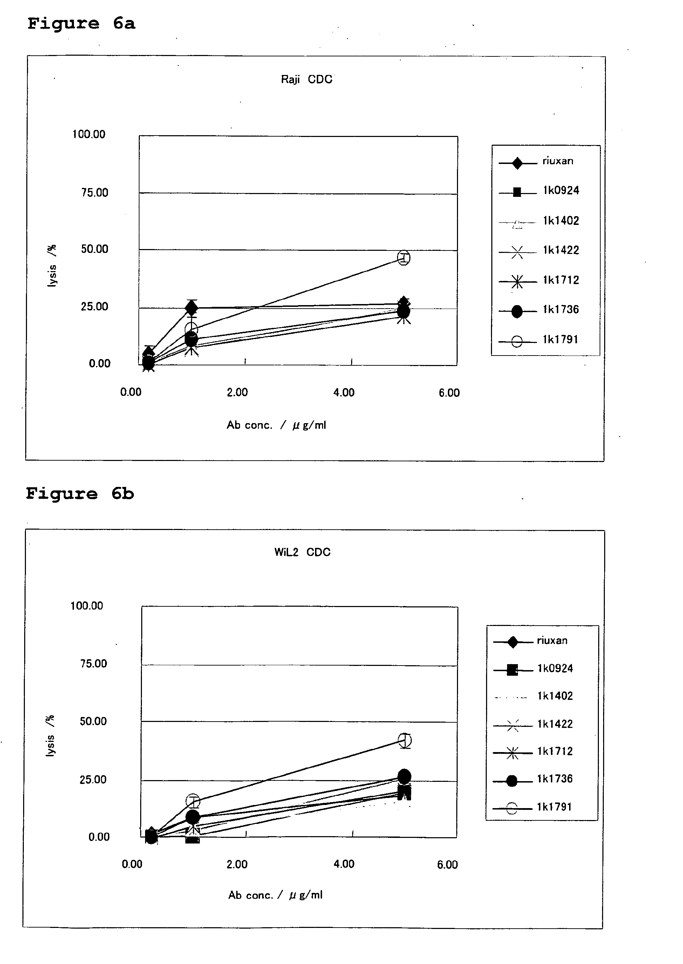

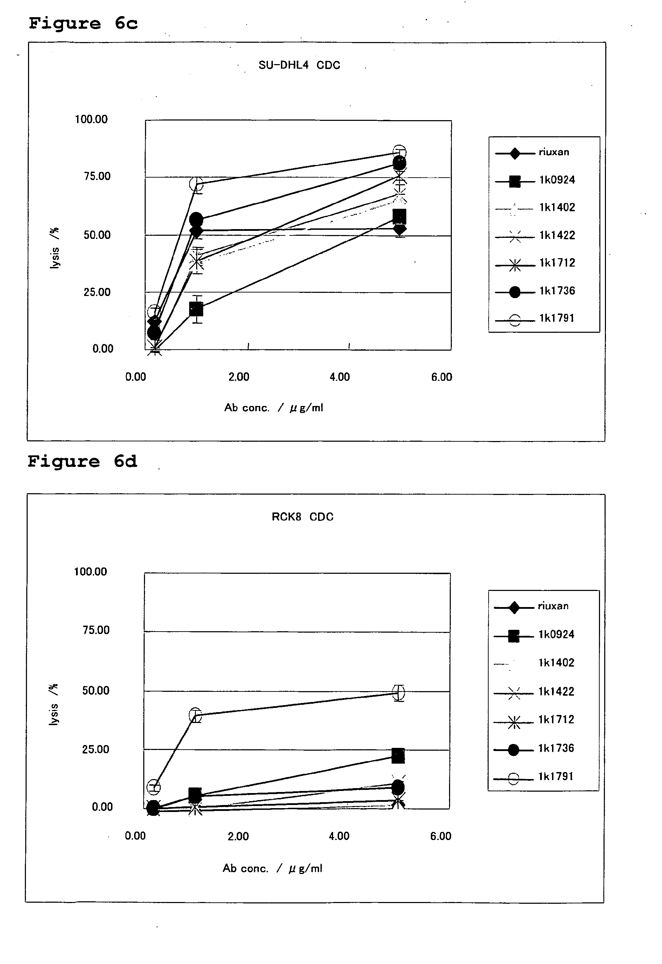

Fig. 6a to Fig. 6d show the relationship between antibody concentration and cytotoxic activity for each cell type.

As shown in Fig. 6a to Fig. 6d, all the six types of antibodies show activity in Raji, WiL2-NS, and SU-DHL4. Furthermore, the concentration dependency can be confirmed, and when compared at a concentration of 5 $\mu$g/ml, 1k1791 shows particularly high activities relative to the other antibodies, followed by 1k1736, 1k1422, and 1k1712 showing high activities. At this concentration, it can be recognized that in Raji and WiL2-NS cells, 1k1791 induces cytotoxicity at levels approaching about two times those induced by the other antibodies, which also display activities equal to or greater than rituximab. RC-K8 cells on which rituximab is considered to have no effect, on the other hand, were found to remarkably show differences between the respective antibodies. Rituximab, 1k1402, and 1k1712 show little or no activity against RC-K8 cells. In contrast, 1k1791 displays very high activity and shows a cytotoxic activity of approximately 50% at a concentration of 5 $\mu$g/ml. Subsequently to 1k1791, 1k0924 shows a cytotoxic activity of 25% or so, and 1k1422 and 1k1736 show a cytotoxic activity of 10% or so.

From the above, it can be confirmed that the 6 types of chimerized antibodies which were used as the subject of these tests display CDC activities equal to or stronger than rituximab.

EXAMPLE 3

(1) Measurement of Binding Affinity

Raji cells derived from human B-cell were cultured in a $CO_2$ incubator at 37°C and at a $CO_2$ concentration of 5% using RPMI 1640 medium supplemented with 10% inactivated FCS. The cells were maintained by subculturing twice a week.

Cell cultures three to four days after subculturing (containing approximately 1 x $10^6$ cells/ml) were centrifuged for five minutes at 1,000 rpm at room temperature to collect the cells. The cells were suspended in PBS(-) and centrifuged for five minutes at 1,000 rpm to remove the supernatant. These procedures were repeated twice for washing the cells.

The reaction with a primary antibody was performed by mixing the respective anti-CD20 antibodies (mouse antibody 2B8 as positive control antibody, chimerized antibodies, and humanized antibody C2B8) with Raji cells and subjecting the mixtures to reaction at room temperature for one hour. In the reaction, the respective anti-CD20 antibodies had twelve final concentrations of 1.33, 2.67, 4.00, 5.33, 6.67, 8.00, 9.33, 10.67, 12.00, 13.33, 14.67, and 16.00 nM, the number of cells was 5x$10^6$ cells, and the reaction solution used a 1% BSA/PBS solution, which was dispensed into 1.5-ml tubes to a final volume of 100 $\mu$l. Three samples for each antibody to be tested were prepared, and as samples for calculation of backgrounds, four tubes to which no antibody was added were also prepared.

After the reaction, the reaction mixtures were centrifuged at room temperature for three minutes at 3,000 rpm to remove any unreacted primary antibody and to collect the cells.

A solution of an FITC-labeled secondary antibody prepared at a concentration of 5 $\mu$g/ml in a 1% BSA/PBS solution was added at a volume of 100 $\mu$l into the tubes, so that the FITC-labeled secondary antibody was in excess with respect to the primary antibody binding to cells, and the mixture, after suspending, was reacted for one hour at room temperature while being shielded from the light.

The FITC-labeled secondary antibodies used were GOAT Anti Mouse IgG (H&L)-FITC for the mouse antibody, and GOAT F(ab') 2F fragment [sic] Anti Human IgG (Fc$\gamma$)-FITC for the chimerized and humanized antibodies.

After the reaction, the mixtures were centrifuged at 3,000 rpm for three minutes at room temperature. Unreacted FITC-labeled secondary antibody was removed and the cells were collected. The cells were washed by suspending them in 200 $\mu$l of PBS and centrifuging them again.

The cells were suspended in 100 $\mu$l of PBS and transferred into flat-bottomed 96-well plates. The intensity of fluorescence of the secondary antibody was measured using a Typhoon 9210 Image Analyzer (Amersham Bioscience).

After the detection, images were digitized using an image analysis software, Image Quant (Amersham Bioscience) and analyzed using Excel (Microsoft). The average value for the same tested antibody was calculated and the average value obtained from the samples for background calculation (in which only the cells and the FITC-labeled secondary antibody were reacted) was subtracted from the value for each of the tested antibodies, thereby to eliminate the background value resulting from the plate, PBS solution, and FITC-labeled secondary antibody binding non-specifically to cells. Additionally, a standard curve was constructed by measuring amounts of the fluorescence from solutions containing only the FITC-labeled secondary antibody in amounts of 0, 12.5, 25, 50, 75, 100, 125, and 150 ng per 100 $\mu$l. This standard curve was used to determine the number of moles of the secondary antibody binding to cells. Assuming that each of the primary antibodies and the FITC-labeled secondary antibody react at a ratio of 1:5, the amount of primary antibody binding to cells was calculated. The amount of free primary antibody was calculated by subtracting the binding amount from the added amount. These values were used for Scatchard analysis, thereby to calculate the dissociation constant Kd.

The results are shown below in Table 4.

(2) Apoptosis Induction Test

Initial apoptosis was detected using a Annexin V-FITC apoptosis kit under two reaction conditions, a first condition where measurements were made of apoptosis which an anti-CD20 antibody alone induced against cells derived from human B-cell stains (non-crosslinking condition), and a second condition where measurements were made of apoptosis which was induced by adding a secondary antibody recognizing the Fc region of an anti-CD20 antibody (crosslinking condition).

Four types of cells, Raji, WIL2-NS, SU-DHL4, and RC-K8 cells derived from human B-cell, were cultured in a $CO_2$ incubator at 37°C and at a $CO_2$ concentration of 5% in RPMI 1640 supplemented with 10% inactivated FCS (culture medium). The cells were maintained by subculturing twice a week.

Three to four days after subculturing, cell cultures (approximately 1 x $10^6$ cells/ml) were centrifuged for five minutes at 1,000 rpm at room temperature to collect the cells.

The respective anti-CD20 antibodies (mouse antibody 2B8 as positive control antibody, chimerized antibodies, and humanized antibody C2B8; Anti-CD2 monoclonal antibody as negative control) were mixed with the cells suspended in fresh culture medium and the mixtures were reacted for 1.5 hours in an incubator at 37°C and at a $CO_2$ concentration of 5%. In the reaction, the respective anti-CD20 antibodies had three final concentrations of 0.2, 1, and 5 $\mu$g/ml, the cell density was 1 x $10^6$ cells/ml, and the reaction solution used culture medium, which was placed in a volume of 250 $\mu$l into 1.5-ml tubes and subjected to reaction. Three samples were prepared for each antibody to be tested.

After the reaction, the mixtures were centrifuged at 1,200 rpm for three minutes at room temperature to remove unreacted antibody and to collect the cells.

Under the non-crosslinking condition, 250 $\mu$l of fresh culture medium was added to the cells. Under the crosslinking condition, 250 $\mu$l of a secondary antibody recognizing the Fc region of the anti-CD20 antibody, which was in an amount of five times the amount of the CD20 antibody, was added to the cells. After mixing, the mixtures were reacted for another three hours in an incubator at 37°C and at a $CO_2$ concentration of 5%. The secondary antibodies used were Goat Anti mouse IgG, Fc$\gamma$ Fragment for the mouse antibody, and Goat Anti human IgG, Fc$\gamma$ Fragment specific, for the chimerized and humanized antibodies.

After the reaction, the mixtures were centrifuged at 3,000 rpm for three minutes at room temperature to remove unreacted secondary antibody and to collect the cells. The cells were washed by suspending them in 250 $\mu$l of PBS and centrifuging them again.

The reagent for detection used a MEBCYTO apoptosis kit-Annexin V-FITC, PI- (MBL, Cat. No. 4700, Lot. 21). After the cells were suspended in 85 μl of Binding buffer, 5 μl of Annexin V-FITC and 5 μl of propidium iodide (PI) (a final concentration of 0.5 mg/ml) were added and mixed well, and the mixture was reacted for 15 minutes at room temperature while being shielded from the light.

20,000 cells in a total count were measured using flow cytometry (EPICS ALTRA: BECKMAN COULTER) and analyzed (Expo32: BECKMAN COULTER).

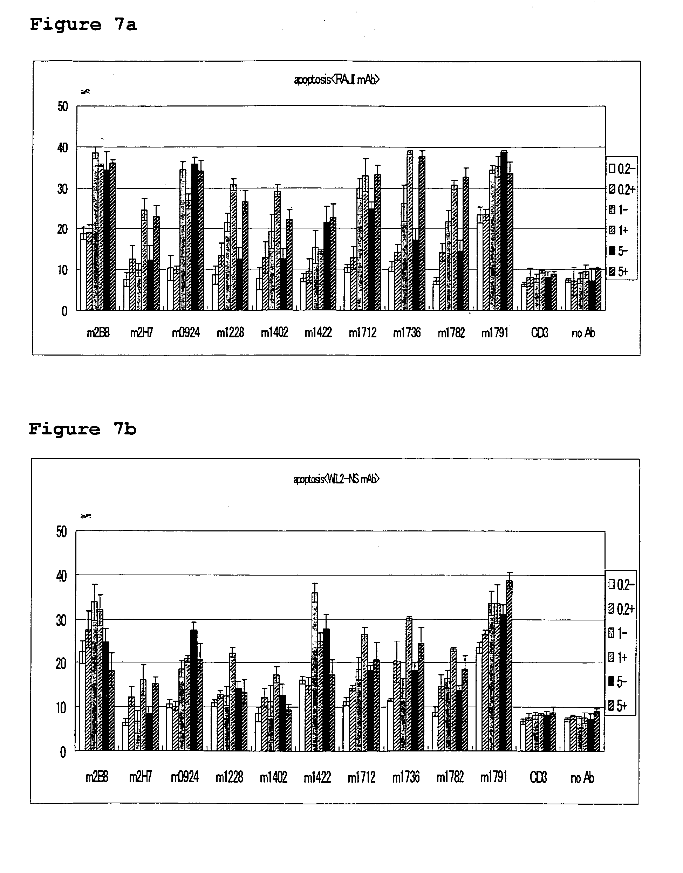

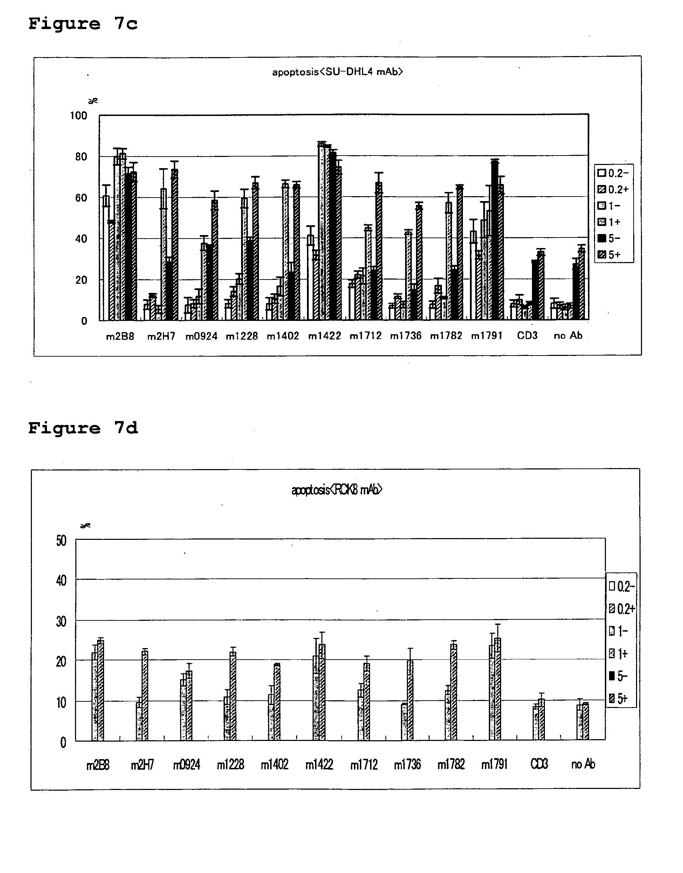

The results are shown in Fig. 7a to Fig. 9d.

(3) Preparation of Humanized Antibody Producing Strains

(a) DNA Synthesis

DNAs with codons optimized for CHO cells, based on the amino acid sequences set forth in SEQ ID Nos: 17 to 24, were designed and synthesized in routine procedures.

(b) Preparation of Constructs

Sixteen types of expression constructs for humanized 1K1791 were constructed using pNOW as an expression vector. Expression constructs for humanized 1K1791 were made. pNOW-aa1791kg1, pNOW-af1791kg1, pNOW-as1791kg1, pNOW-av1791kg1, pNOW-fa1791kg1, pNOW-ff1791kg1, pNOW-fs1791kg1, pNOW-fv1791kg1, pNOW-sa1791kg1, pNOW-sf1791kg1, pNOW-ss1791kg1, pNOW-sv1791kg1, pNOW-va1791kg1, pNOW-vf1791kg1, pNOW-vs1791kg1, pNOW-vv1791kg1.

(c) Transfection and Selection Using Chemical Reagents

The respective expression constructs for humanized 1K1791 were introduced into CHO DG44cdB cells using a transfection reagent. $1 \times 10^6$ CHO DG44cdB cells into which each of the genes was introduced were suspended in 100 ml of selective medium, seeded on five 96-well plates (200 μl/well), and cultured for 3 to 4 weeks at 37°C under an atmosphere of 5% carbon dioxide gas.

Transfection reagent: Qiagen, Effectene Transfection Reagent, Cat. No 301427.

Selective Medium: IS CHO-CD w/Hydrolysate/4mM GlutaMAX/0.8 mg/ml G418.

(d) Selection of High Expression Cell Strains

1) Supernatants were recovered from wells where colonies were present and the amount of antibody produced was measured using a Dot Blot assay.
2) Clones displaying high amounts of antibody produced were transferred into 24-well plates and after culturing for approximately 5 days, the supernatants were recovered and the amount of antibody produced was measured using Sandwich ELISA
3) Two clones displaying high levels of expression were selected and transferred into T75 flasks.

(e) Small Scale Culturing

Two clones selected for each of the 16 types of constructs were cultured in T75 flasks containing 30 ml of the selective medium.

(4) Culturing of Humanized Antibody Producing Strains and Purification

A antibody producing cell strain (genetically recombinant CHO-DG44 cells) was cultured in Hydrolysate-containing IS CHO-CD/with Hydrolysate medium (Irvine Scientific, Cat. No. 91119) supplemented with 4 mM GlutaMax (Invitrogen, Cat 35050-061) and 200 μg/ml of G418 (Sigma, Cat. No. A1720-5G) in a $CO_2$ incubator at 37°C and at a $CO_2$ concentration of 5%. The cells were maintained by subculturing twice a week.

Cell culture about two weeks after subculturing was started was centrifuged at 3,500 rpm for five minutes at room temperature to collect the supernatant, which in turn was filtered using a 0.45-μm syringe filter and equilibrated with 50 mM Tris-HCl, pH 7.0.

The supernatant was loaded onto a Hi Trap Protein A HP column (GE Healthcare, Cat No. 17-0402-01), and then washed using 50 mM Tris-HCl, pH 7.0. Elutions were obtained using 0.1 M citric acid, pH 4.0. Fractions of 400 μl were collected

and neutralized with 40 μl, corresponding to 10/1 [sic] of the fraction volume, of 1 M Tris-HCl, pH 9.0. Dialysis was performed against PBS of 100-times volume using Slyde-A Lyzer 10K Dialysis Cassetes (PIERCE, Cat. No. 66453) twice for a period of 2.5 hours each, then once for a period of 15 to 18 hours. The purified sample was filtered using a 0.22-μm syringe filter, followed by concentration using a VIVA SPIN 50,000 MWCO PES (VIVASCIENCE, Cat. No. VS0231). The antibody concentration was calculated from the A280 value using a BECKMAN COULTER DU530.

The antibody producing strains used were CHO cells hz1791-fv10, hz1791-ff34, hz1791-sf43, and hz1791-ss32, which were deposited under Accession Nos. FERM BP-10543, FERM BP-10544 FERM BP-10545, and FERM BP-10546, respectively, with the International Patent Organism Depositary, National Institute of Advanced Industrial Science and Technology, Tsukuba Central 6, 1-1-1 Higashi, Tsukuba, Ibaraki, Japan, on 1 March in the 18th year of Heisei (2006), under the provisions of the Budapest Treaty.

The amino acid sequences of the H-chain V-region and the L-chain V-region of humanized anti-CD20 monoclonal antibodies obtained from these cell strains (SEQ ID Nos: 17 to 24) are described hereinafter (the underlined amino acids differ from those of the corresponding mouse antibodies).

Sequences for Humanized 1k1791:

The sequence of the L-chain V-region (SEQ ID No: 20): Ven 1791: STVMTQSPDSLAVSLGERVTINC KASQSVSNDVA WYQQKPGQSPKVLIY FASNRYT GVPDRFSGSGYGTDFTFTISSVQAEDVAVYFC QQDYSSPLT FGAGTKLELK

The sequence of the H-chain V-region (SEQ ID No: 24): Ven 1791: QIQLVQSGPELKKPGASVKISCKASGYTFT NFGVN WVKQAPGKGLKWMG WINTYTGEPSYADDFKG RFAFSLDASVSTAY LQISSLKAEDTSTYFCTR RTNYYGTSYYYAMDY WGQGTTVTVSS

The sequence of the L-chain V-region (SEQ ID No: 17): abb 1791: STVMTQSPDSLAVSLGERATINC KSSQSVSNDVA WYQQKPGQSPKVLIY FASNRYS GVPDRFSGSGYGTDFTLTISSLQAEDVAVYFC QQDYSSPLT FGAGTKLEIK

The sequence of the H-chain V-region (SEQ ID No: 21): abb 1791: QIQLVQSGSELKKPGASVKVSCKASGYTFT NFGVN WVRQAPGKGLEWMG WINTYTGEPSYAQGFTG RFVFSLDASVSTAY LQISSLKAEDTATYFCTR RTNYYGTSYYYAMDY WGQGTTVTVSS

The sequence of the L-chain V-region (SEQ ID No: 19): sdr 1791: STVMTQSPDSLAVSLGERATINC KSSQSNSNDVA WYQQKPGQSPKVLIY FASNRYS GVPDRFSGSGYGTDFTLTISSLQAEDVAVYFC QQDYSSPLT FGAGTKLEIK

The sequence of the H-chain V-region (SEQ ID No: 23): sdr 1791 QIQLVQSGSELKKPGASVKVSCKASGYTFT NFGVN WVRQAPGKGLKWMG WINTYTGEPSYAQGFTG RFAFSLDASVSTAY LQISSLKAEDTATYFCTR RTNYYGTSYYYAMDY WGQGTTVTVSS

The sequence of the L-chain V-region (SEQ ID No: 18): fra 1791: STVMTQSPSFLSASVGDRVTITC KASQSVSNDVA WYQQKPGQSPKVLIY FASNRYT GVPDRFSGSGYGTDFTLTISSLQAEDVAVYFC QQDYSSPLT FGAGTKLEIK

The sequence of the H-chain V-region (SEQ ID No: 22): fra 1791: QIQLVQSGSELKKPGASVKVSCKASGYTFT NFGVN WVKQAPGKGLKWMG WINTYTGEPSYADDFKG RFAFSLDASASTAY LQISSLKAEDMATYFCTR RTNYYGTSYYYAMDY WGQGTTVTVSS

In this series of experiments, experiments were performed with respect to two clones derived from each of a total of 16 sequence-combinations resulting from combining four types which were AbbL, FraL, SdrL, and VenL, and four types which were AbbH, FraH, SdrH, and VenH. From the above results, these clones can be classified into 4 groups as shown in Table 5, based on their Kd values. One clone was selected from each of the groups and used in further experiments.

In the specification, for example, when clone fv of humanized antibody 1791 is referred to, it means an antibody clone in which the L-chain of fra1791 (the L-chain set forth in SEQ ID No: 18) and the H-chain of Ven1791 (the H-chain set forth in SEQ ID No: 24) are combined (underlining is done for explanation). This similarly applies to other clones.

Control: c2B8
Group I: Kd = 20 nM <
Group II: Kd = 10 to 20 nM
Group III: Kd = 8 to 10 nM
Group IV: Kd = <8 nM

Binding Dissociation Constants Of Humanized Antibodies

Table 4

| Hz1791 clone No. | Kd (nM) | C2B8 Kd (nM) |
|---|---|---|
| Aa008 | 11.68 | 6.61 |
| Aa012 | 9.96 | 4.68 |
| Fa007 | 11.34 | 5.63 |
| Fa008 | 8.83 | 4.91 |
| Sa023 | 14.66 | 6.13 |
| Va016 | 13.09 | 6.67 |
| Va024 | 7.50 | 6.47 |
| Af021 | 6.84 | 5.56 |
| Af025 | 8.67 | 4.29 |
| Ff019 | 8.50 | 5.70 |
| Ff034 | 7.81 | 4.00 |
| Sf043 | 11.60 | 4.34 |
| Sf056 | 13.79 | 6.01 |
| Vf029 | 7.78 | 3.32 |
| Vf031 | 7.79 | 4.54 |
| As001 | 11.89 | 5.74 |
| As002 | 11.47 | 8.7 |
| Fs007 | 6.33 | 5.63 |
| Fs024 | 11.09 | 3.97 |
| Ss020 | 24.39 | 4.10 |
| Ss032 | 21.79 | 7.25 |
| Vs006 | 8.8 | 4.23 |
| Vs011 | 11.9 | 6.09 |
| Av004 | 10.71 | 5.76 |
| Av006 | 9.17 | 4.86 |
| Fv010 | 7.17 | 4.39 |
| Fv028 | 7.25 | 4.74 |
| Sv015 | 14.31 | 6.30 |
| Sv020 | 10.85 | 5.36 |
| Vv018 | 7.14 | 5.04 |
| Vv023 | 7.01 | 4.86 |

Binding Dissociation Constants Of Humanized Antibodies

Table 5

| Group | Hz1791 clone | Kd (nM) |
|---|---|---|
| I (20 nM <) | ss | 23.09 |

(continued)

| Group | Hz1791 clone | Kd (nM) |
|---|---|---|
| II (10 to 20 nM) | sa | 14.66 |
| | sf | 12.70 |
| | sv | 12.58 |
| | as | 11.68 |
| | aa | 10.82 |
| | vs | 10.35 |
| | va | 10.30 |
| | fa | 10.09 |
| III (8 to 10 nM) | av | 9.94 |
| | fs | 8.71 |
| | ff | 8.15 |
| IV (< 8 nM) | vf | 7.78 |
| | af | 7.76 |
| | fv | 7.21 |
| | vv | 7.07 |
| Control | c2B8 | 5.35±1.13 |

The results of these experiments for apoptosis using 8 types of mouse antibodies are shown in Fig. 7a to Fig. 7d. The inventors were able to divide the 8 types of mouse antibodies and known CD20 antibodies, 2B8 and 2H7, into two groups with respect to all the four types of cells (some exceptions were not included).

Group A: m0924, m1422, m1791, m2B8
Group B: m1228, m1402 m1712, m1782, m2H7

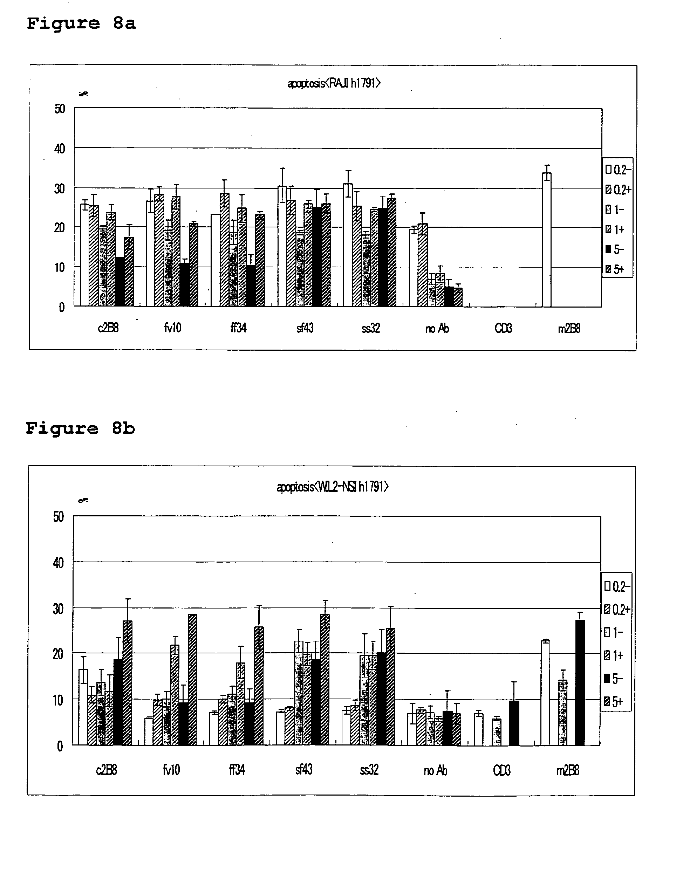

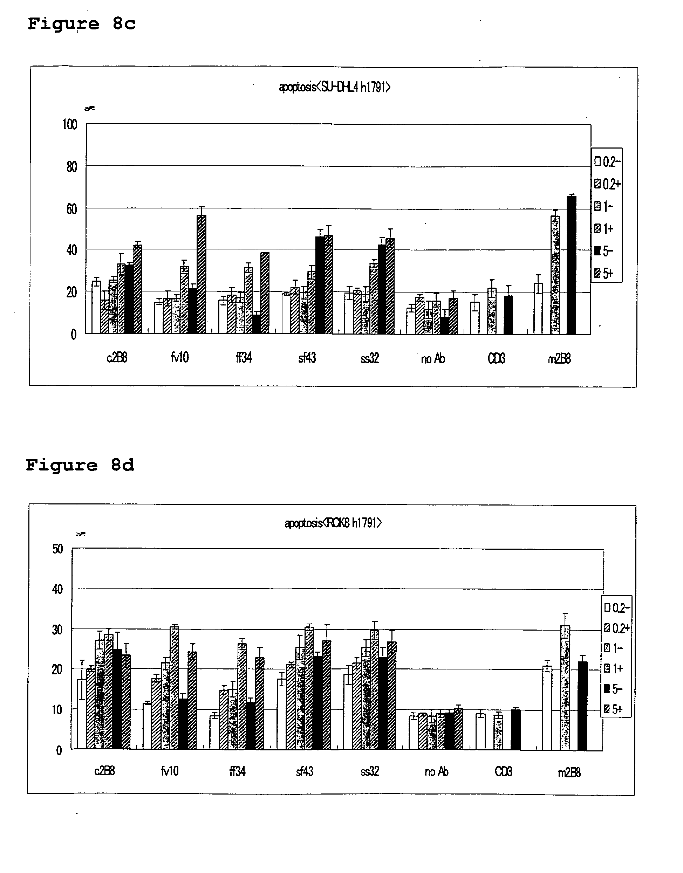

(However, m0924 was included in Group B with respect to SU-DHL4 cells).
In other words, Group A includes anti-CD20 antibodies which display sufficient capabilities to induce apoptosis by themselves and have approximately the same level of apoptosis inducing capability even under the crosslinking condition with a secondary antibody, while Group B includes anti-CD20 antibodies which display insufficient capabilities to induce apoptosis by themselves and have an greatly increased capabilities to induce apoptosis under the crosslinking condition. Furthermore, the antibodies belonging to Group A display affinities comparable to that of 2B8, while the antibodies belonging to Group B display higher affinities than that of 2B8. Consequently, it can be inferred that the antibodies of Group A are more suitable for pharmaceutical use since they do not require the presence of a secondary antibody to induce apoptosis and are capable of inducing apoptosis by themselves.
Turning to the type of cells, the results indicate that RAJI, WIL2-NS, RCK8 cells had ratios of apoptosis, at highest, in the range of 30 to 40%, whereas SU-DHL4 cells displayed maximum ratios of not less than 80% in some cases.
The results for the four types of humanized antibodies are shown in Fig. 8a to Fig. 9d.
Similarly to the mouse antibodies, four clones of humanized antibody 1791 were classified into two groups with respect to all the four types of cells.
Group A: fv10, ff34
Group B: sf43, ss32, c2B8
Antibodies fv10 and ff34 of Group A, which display comparable affinities to that of C2B8, have almost the same apoptosis activity as that of C2B8 under the non-crosslinking condition and clearly increased activities under the crosslinking condition. Although antibodies sf and ss of Group B display greater dissociation constants and weaker affinities than the antibodies of Group A, these antibodies alone display slightly stronger apoptosis activities than C2B8.
As concerns the capability of the anti-CD20 antibodies to induce apoptosis against B-cells, when they display sufficient capabilities of inducing apoptosis by themselves, the ratio of apoptosis is substantially the same under the crosslinking condition. However, when they do not display a sufficient capability of inducing apoptosis by themselves, due to the type of antibodies, unsaturated conditions, or the like, it is likely that apoptosis activity will increase under the crosslinking condition.

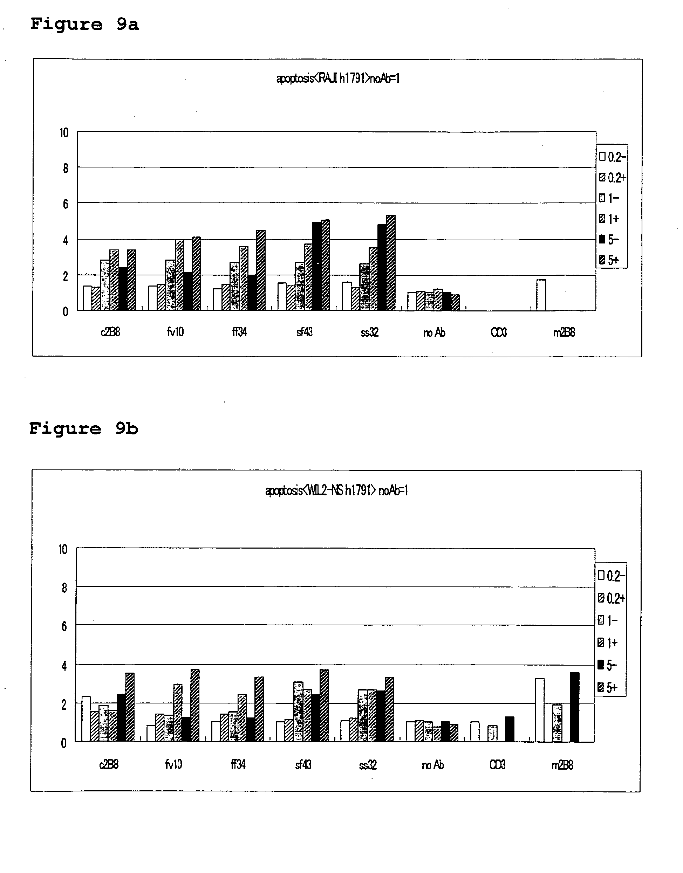

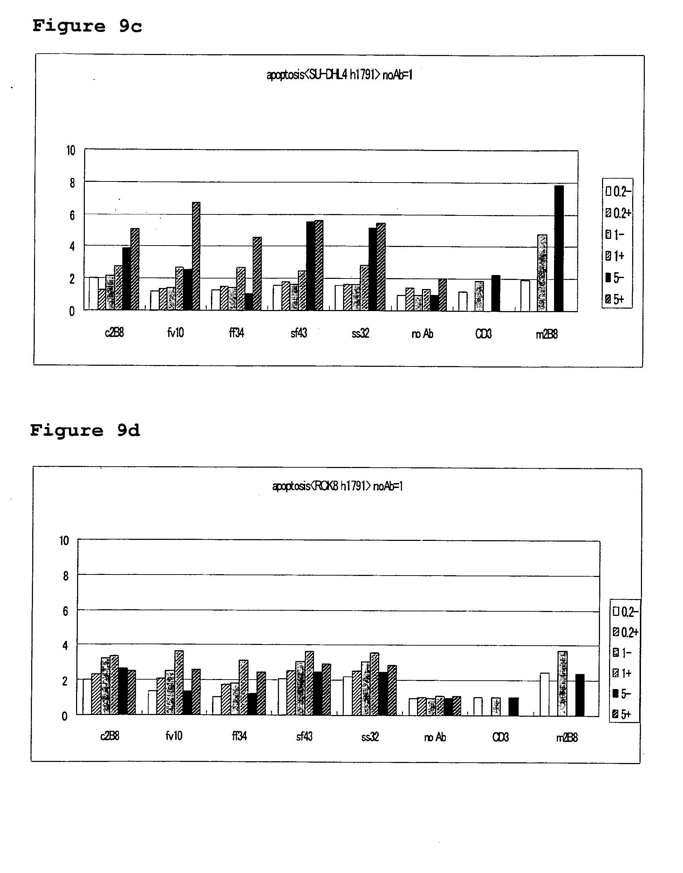

Fig. 9a to Fig. 9d are graphs showing early apoptosis (%) wherein the ratio of early apoptosis under conditions of "no Ab" (no added antibody) on the day of the experiments is set to 1.

Apoptosis activities of four clones of humanized antibody 1791, clones fv, ff, sf, and ss, against Raji, SU-DHL4, WiL-2, and RCK8 cells are summarized in Table 6.

Summary of Apoptosis Activities of Humanized Antibody-1791 Clones

Table 6

| Antibody | Affinity (average) | Apoptosis (5 ug/ml) | | | | | | | |
| | Raji | RAJI | | Wi12-NS | | DHL4 | | RCK8 | |
| | Kd (nM) | m2B8=100 | XL w/wo | m2B8=100 | XL w/wo | m2B8=100 | XL w/wo | m2B8=100 | XL w/wo |
|---|---|---|---|---|---|---|---|---|---|
| c2B8 | 5.35 | 100 | 1.4 | 100 | 1.4 | 100 | 1.3 | 100 | 0.9 |
| fv | 7.21 | 88 | 1.9 | 49 | 3.1 | 65 | 2.7 | 50 | 1.9 |
| ff | 8.15 | 84 | 2.2 | 49 | 2.8 | 96 | 1.2 | 47 | 1.9 |
| sf | 12.70 | 205 | 1.0 | 100 | 1.5 | 143 | 1.0 | 93 | 1.2 |
| ss | 23.09 | 202 | 1.1 | 108 | 1.3 | 133 | 1.1 | 92 | 1.2 |
| no Ab | | 42 | 0.9 | 41 | 0.9 | 26 | 2.0 | 37 | 1.1 |

CDC activities were measured for the clones fv, ff, sf, and ss, of humanized antibody 1791 using Raji, SU-DHL4, WiL-2, and RCK8 cells. The respective results are shown in Table 7. Rituximab (c2B8) and the clone 1791 (c1791) were included as controls.

CDC Activities of Humanized Antibodies according to the Present Invention

Table 7

| Antibody | CDC (10 ug/ml) | | | |
|---|---|---|---|---|
| | RAJI | WIL2NS | SUDHL4 | RC-K8 |
| | c2B8=100 | c2B8=100 | c2B8=100 | c2B8=100 |
| c2B8 | 100 | 100 | 100 | 3 |
| fv | 99 | 172 | 120 | 498 |
| ff | 98 | 203 | 121 | 530 |
| sf | 78 | 295 | 111 | 713 |
| ss | 73 | 120 | 61 | 108 |
| c1791 | 99 | 529 | 119 | 100 |

The results in Table 7 show that the clones ff and fv displayed CDC activities which were equal to or stronger than that of rituximab. Furthermore, sf showed extremely strong CDC activities with respect to WiL2 and RCK8 cells.

Experiments were also-conducted to investigate the relationship between antibody concentration and CDC activity. The antibodies used were purified to a higher degree of purification than that of the antibodies prepared as described in Example 2. Purification was carried out in the procedures described below.

A antibody producing cell strain (genetically recombinant CHO-DG44 cells) was cultured in Hydrolysate-containing IS CHO-CD/w medium (Irvine Scientific, Cat. No. 91119) supplemented with 4 mM GlutaMax (Invitrogen, Cat. 35050-061) and 200 $\mu$g/ml of G418 (Sigma, Cat. No. A1720-5G) in a $CO_2$ incubator at 37°C and at a $CO_2$ concentration of 5%. The cells were maintained by subculturing twice a week. Cell culture approximately two weeks after subculturing was centrifuged at 3,500 rpm for five minutes at room temperature to collect the supernatant, which in turn was filtered using a 0.45-$\mu$m syringe filter and equilibrated with 50 mM Tris-HCl, pH 7.0. The supernatant was loaded onto a Hi Trap Protein A HP column (GE Healthcare, Cat. No. 17-0402-01), and then washed using 50 mM Tris-HCl, pH 7.0. Elutions were obtained using 0.1 M citric acid, pH 4.0. Fractions of 400 $\mu$l were collected and neutralized with 40 $\mu$l, corresponding 10/1 [sic] of the fraction volume, of 1 M Tris-HCl, pH 9.0. Dialysis was performed against PBS of 100-times volume using Slyde-A-Lyzer 10K Dialysis Cassetes (PIERCE, Cat. No. 66453) twice for a period of 2.5 hours each, then once for a period of 15 to 18 hours.

The dialyzed sample was concentrated using a VIVASPIN 50,000 MWCO PES (VIVASCIENCE, Cat. No. VS0231). The sample was loaded onto a HiLoad 16/60 superdex 200 prep grade column (GE Healthcare Cat No. 17-1069-01) equilibrated with PBS. The purified sample was filtered using a 0.22-$\mu$m syringe filter and concentrated using a VIVASPIN 50,000 MWCO PES (VIVASCIENCE, Cat. No. VS0231). The antibody concentration was calculated from the A280 value using a BECKMAN COULTER DU530.

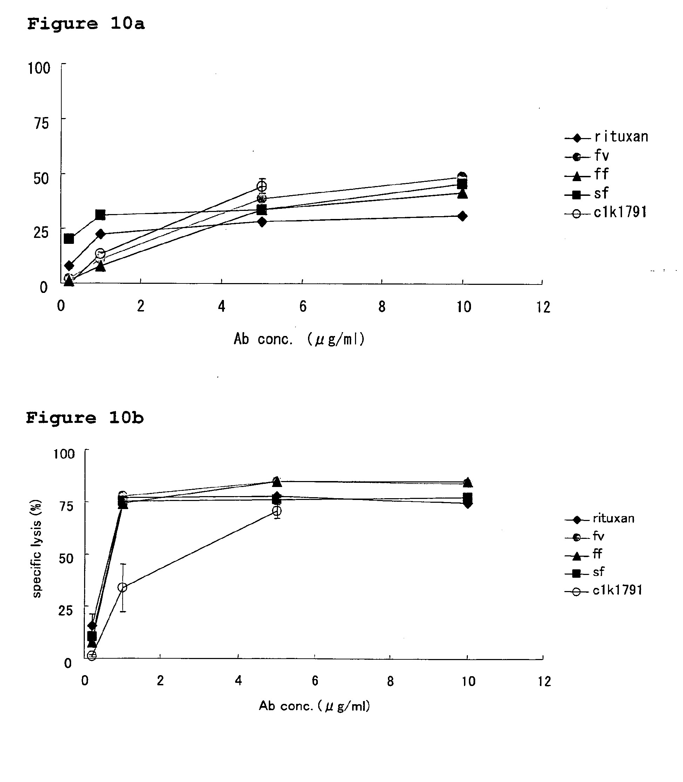

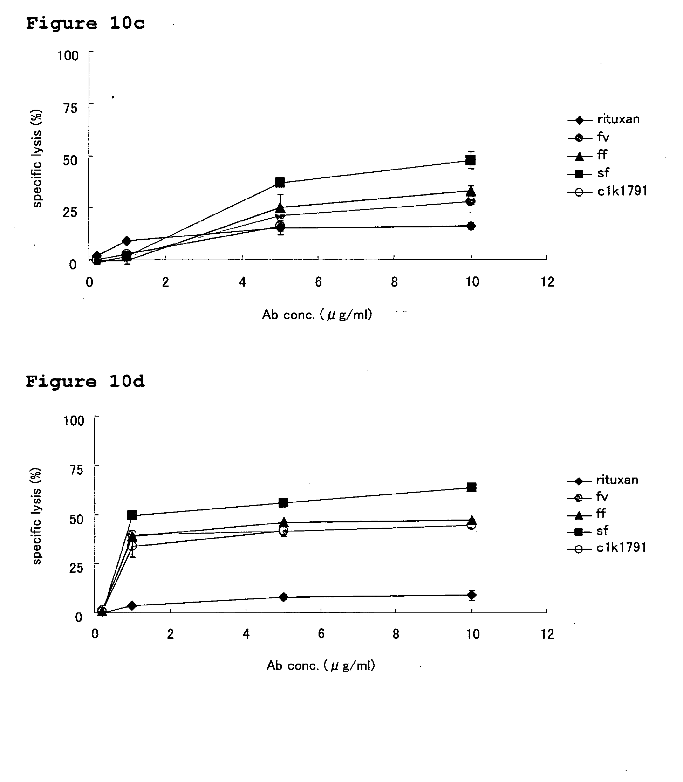

Fig. 10a to Fig. 10d show the relationship between concentration and CDC activity of the humanized antibodies fv, ff, sf, and ss, and the chimerized antibody c1k179, for Raji, SU-DHL4, WiL-2, and RCK8 cells. In all the experiment systems, CDC activities of the humanized antibodies fv, ff, sf, and ss at a concentration of 5 $\mu$g/ml or more were equal to or greater than that of Rituxan (C2B8). In particular, although Rituxan displayed little CDC activity against RC-K8 cells, the four clones of humanized antibody 1791 according to the present invention displayed CDC activities against RC-K8 cells at least about 4 times higher than Rituxan.

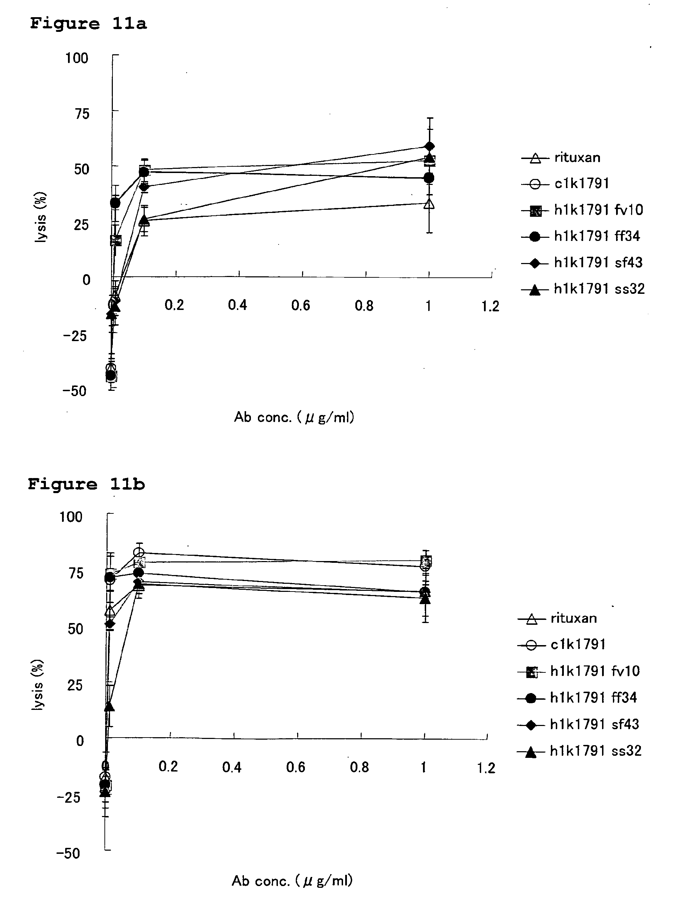

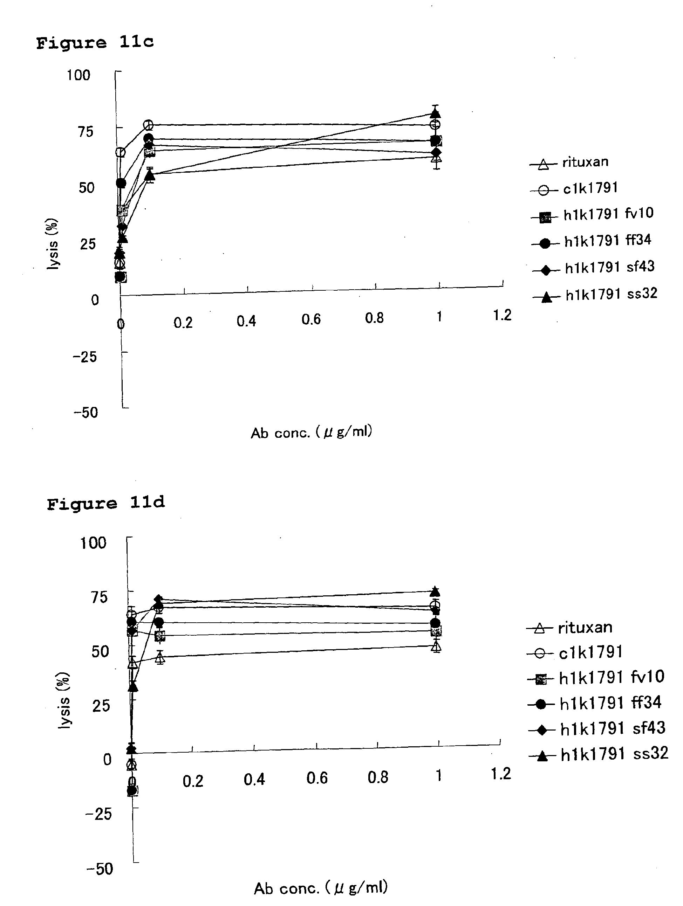

The ADCC activity of fv, ff, sf, and ss against Raji, SU-DHL4, WiL-2, and RCK8 cells was also examined. The relationship between antibody concentration and ADCC is shown in Fig. 11a to Fig. 11d (the E:T ratio was 25). ADCC activities of four clones fv, ff, sf, and ss of humanized antibody 1791 according to the present invention were equal to or greater than that of Rituxan (C2B8). The results for these ADCC activities also demonstrate the efficacy of the series 1791 of humanized antibodies according to the present invention.

EXAMPLE 4

Obtaining of Another Type of Humanized Anti-CD20 Monoclonal Antibody (Humanized Antibody 1782)
The series 1782 was selected as antibodies having highest affinities from the group of antibodies which do not induce apoptosis by themselves and have high affinity, and subjected to humanization. Clones of humanized antibody 1782 were obtained and their properties were examined in accordance with the procedures described in the previous Examples. Properties of the resulting humanized antibodies were also were examined in accordance with the procedures described in the previous Examples.

DNAs with codons optimized for CHO cells, based on amino acid sequences in SEQ ID Nos: 27 to 34, were designed and synthesized in routine procedures. Sixteen types of expression constructs for humanized 1K1782 were constructed using pNOW as an expression vector. These constructs were as follows:

pNOW-aa1782kg1, pNOW-af1782kg1, pNOW-as1782kg1, pNOW-av1782kg1, pNOW-fa1782kg1, pNOW-ff1782kg1, pNOW-fs1782kg1, pNOW-fv1821kg1, pNOW-sa1782kg1, pNOW-sf1821kg1, pNOW-ss1782kg1, pNOW-sv1782kg1, pNOW-va1782kg1, pNOW-vf1782kg1, pNOW-vs1782kg1, and pNOW-vv1782kg1.

In addition, transfection and selection using a chemical reagent were performed in accordance with the procedures described in Example 3, for selection of high expression cell strains, small scale culturing, and culturing of humanized antibody producing strains and purification of humanized antibodies.

The antibody producing strains used were CHO cells hz1782-ss21, hz1782-fv02, hz1782-ff14, and hz1782-sf23, of which the strains hz1782-ss21, hz1782-fv02, and hz1782-ff14 were deposited under Accession Nos. FERM ABP-10907, FERM ABP-10906, and FERM ABP-10905, respectively, with the International Patent Organism Depositary, National Institute of Advanced Industrial Science and Technology, Tsukuba Central 6, 1-1-1 Higashi, Tsukuba, Ibaraki, Japan, on 31 August in 19th year of Heisei (2007) under the provisions of the Budapest Treaty.

The amino acid sequences of the H-chain V-region and the L-chain V-region of humanized anti-CD20 monoclonal antibodies obtained from these cell strains (SEQ ID Nos: 17 to 24) are described hereinafter.

Sequences for Humanized 1k1782:

The sequence of the L-chain V-region (SEQ ID No: 27): Ven 1782: DILLTQSPATLSLSPGERATLSC RASQNIGTSIH WYQQKPGQSPRLLIK YASESFS GIPSRFSGSGSGTDFTLTISSLEPEDFADYYC QQSNSWPFT FGSGTKLEIK

The sequence of the H-chain V-region (SEQ ID No: 31): Ven 1782: QVQLVQSGAELKKPGASVKVSCKAS-GYTFT SYWMH WVKQAPGQGLEWIG YITPSTGYTDYNKKFKD KATLTADRSSSTAYMELSSLRSEDTAVYY-CAR SGPYFDV WGAGTTVTVSS

The sequence of the L-chain V-region (SEQ ID No: 28): abb 1782: DIVLTQSPATLSLSPGERATLSC RASQNIGTSIH WYQQKPGQSPRLLIK YASESFT GIPSRFSGSGSGTDFTLTISSLEPEDFADYYC QQSNSWPFT FGSGTKLEIK

The sequence of the H-chain V-region (SEQ ID No: 32): Abb 1782: QVQLVQSGAEVKKPGASVKVSCKAS-GYTFT SYWMH WVRQAPGQGLEWMG YITPSTGYTDYNQKFQG RVTLTADRSSSTAYMELSSLRSED-TAVYYCAR SGPYFDV WGAGTTVTVSS

The sequence of the L-chain V-region (SEQ ID No: 29): sdr 1782: DILLTQSPATLSLSPGERATLSC RASQNVGTSLH WYQQKPGQSPRLLIK YASERFT GIPSRFSGSGSGTDFTLTISSLEPEDFADYYC QQSNSWPFT FGSGTKLEIK

The sequence of the H-chain V-region (SEQ ID No: 33): sdr 1782: QVQLVQSGAEVKKPGASVKVSCKASGYTFT SYWMH WVKQAPGQGLEWIG YITPSTGYTDYNQKFQG KATLTADRSSSTAYMELSSLRSEDTAVYYCAR SGPYFDV WGAGTTVTVSS

The sequence of the L-chain V-region (SEQ ID No: 30): fra 1782 : DILLTQSPATLSVSPGERATLSC RASQNIGTSIH WYQQRPGQSPRLLIK YASESFS GIPSRFSGSGSGTDFTLTINSLQPEDIADYYC QQSNSWPFT FGSGTKLEIK

The sequence of the H-chain V-region (SEQ ID No: 34): fra 1782: QVQLVQSGAEVKKPGASVKVSCKASGYTFT SYWMH WVKQAPGQGLEWIG YITPSTGYTDYNKKFKD KATLTADRSSSTAYMELSSLRSEDTAVYYCAR SGPYFDV WGAGTTVTVSS

In the specification, when clone fv of humanized antibody 1782 is referred to, it means an antibody clone in which the L-chain of fra1782 (the L-chain set forth in SEQ ID No: 30) and the H-chain of Ven1782 (the H-chain set forth in SEQ ID No: 31) are combined. This similarly applies to other clones.

(a) Measurement of Affinity of Humanized Antibody 1782

With reference to the results in Example 3, the inventors made the assumption that clones of humanized antibody 1782 are also classified into four groups, and selected ss, sf, ff, and fv belonging to the respective groups. Kd values against CD20 were measured for these antibodies, Rituxan (c2B8), and humanized antibody 2f2. The results are shown in Table 8.

In the specification, for example, when clone sf of humanized antibody 1782 is referred to, it means an antibody clone in which the L-chain of sdr1782 (the L-chain set forth in SEQ ID No: 29) and the H-chain of fra1782 (the H-chain set forth in SEQ ID No: 34) are combined (underlining is done for explanation). This similarly applies to other clones.

Binding Dissociation Constants (Kd) of Humanized Antibody-1782 Clones

Table 8

| Clone | c2B8 | 2f2 | h1782ff | h1782fv | h1782sf | h1782ss |
|---|---|---|---|---|---|---|
| Kd (nM) | 5.43±0.93 | 5.26 | 4.38 | 6.76 | 7.43 | 8.64 |

The Kd values of these humanized antibodies were examined in accordance with the previous Examples. The cell used was Raji cell. Detection of the primary antibody used FITC-labeled Protein A, and analysis was made on the assumption that the binding ratio of primary antibody and Protein A is 1:3.

The clone ff of humanized antibody 1782 displayed the lowest Kd value, which was lower than those of Rituxan and humanized antibody 2f2. That is, it was found that the clone ff of humanized antibody 1782 has the highest affinity to CD20.

(b) Cell Growth Inhibiting Activity of Humanized Antibody 1782

Four clones fv, ff, sf, and ss of humanized antibody 1782 were measured for apoptosis inducing activity, CDC activity (%), and ADCC activity. These activities were measured in accordance with the procedures described in the previous Examples.

(b1) Apoptosis Inducing Activity of Humanized Antibody 1782

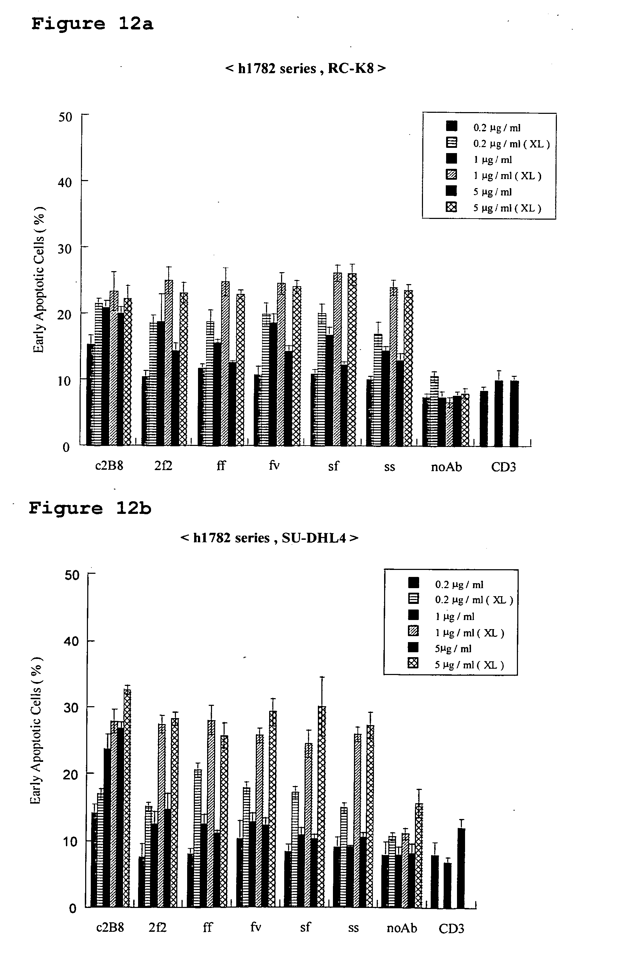

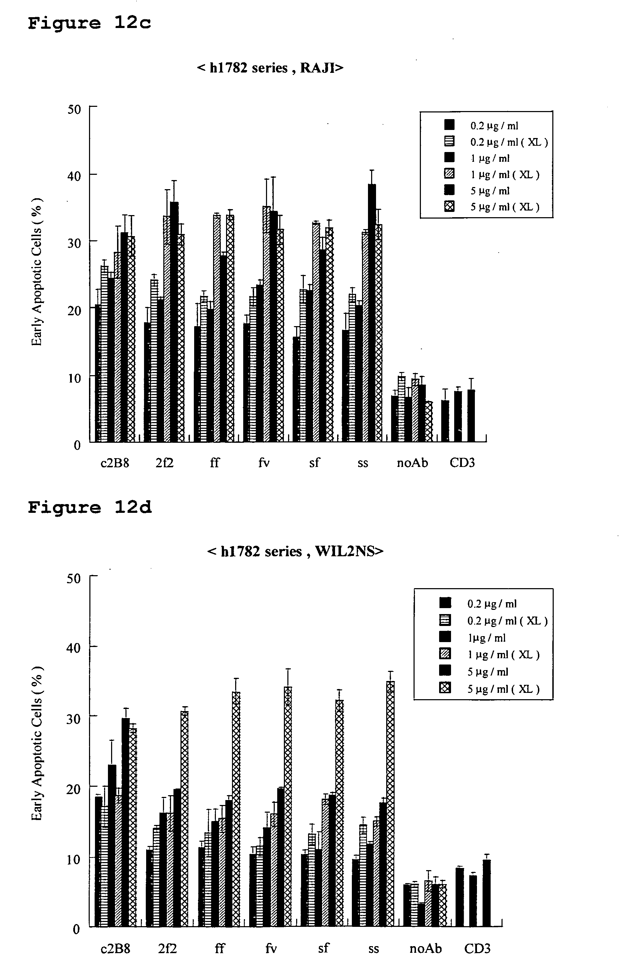

The apoptosis inducing activity of humanized antibody 1782 was measured using RC-K8, SU-DHL4, RAJI, and WIL2NS cells. The results are shown in Fig. 12a to Fig. 12d (in the figures), the numbers on the right side represent the antibody concentration, and XL represents the presence of crosslinking with a secondary antibody (goat anti-human antibody).

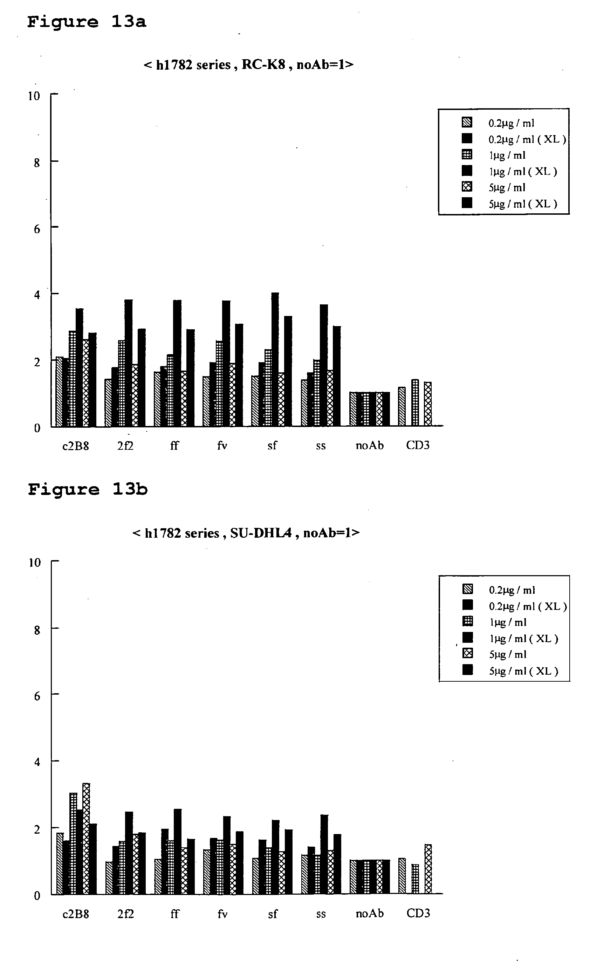

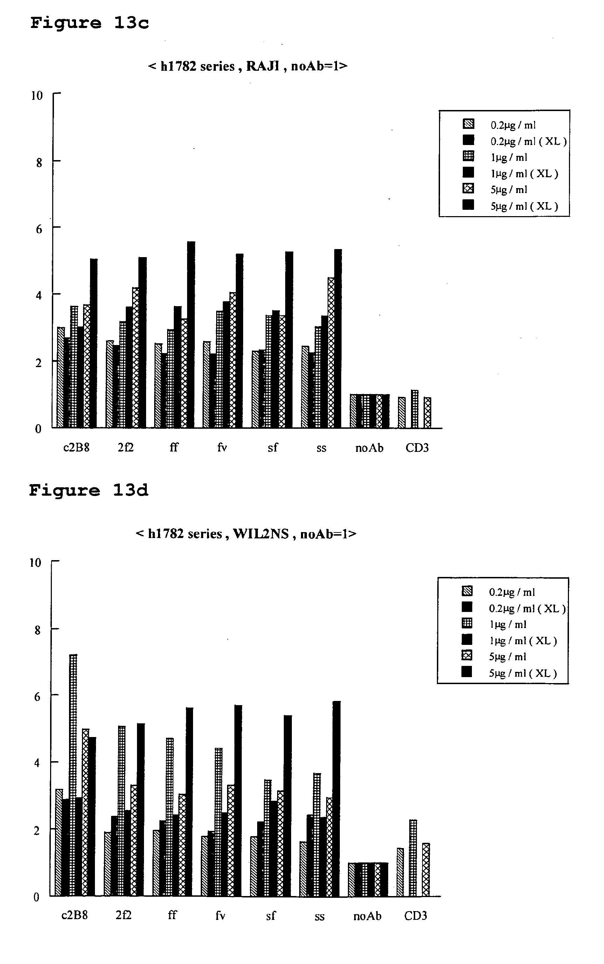

Fig. 13a to Fig. 13d show apoptosis inducing activities of humanized antibody-1782 clones, when the capability of inducing apoptosis under conditions with no added antibody is set to 1.

The series 1782 of humanized antibodies displayed weak activities of inducing apoptosis by themselves and apoptosis inducing activities comparable to or greater than that of Rituxan (c2B8) under the crosslinking condition. The results are summarized in Table 9.

Apoptosis Inducing Activities of Humanized Antibody-1782 clones (in the table, XL represents the presence of crosslinking with a secondary antibody (goat anti-human antibody).

Table 9

| Clone | Affinity (average) | Apoptosis (5 ug/ml) | | | | | | | |
|---|---|---|---|---|---|---|---|---|---|
|  | Raji | RAJI | | WIL2NS | | SUDHL4 | | RC-K8 | |
|  | Kd (nM) | c2B8 =100 | XL w/wo | c2B8 =100 | XL w/wo | c2B8 =100 | XL w/wo | c2B8 =100 | XL w/wo |
| c2B8 | 5.43±0.93 | 100 | 1.0 | 100 | 1.0 | 100 | 1.2 | 100 | 1.1 |
| 2f2 | 5.26 | 115 | 0.9 | 66 | 1.6 | 54 | 1.9 | 72 | 1.6 |
| ff | 4.38 | 89 | 1.2 | 63 | 1.9 | 41 | 2.3 | 63 | 1.8 |
| fv | 6.76 | 110 | 0.9 | 61 | 1.7 | 46 | 2.4 | 72 | 1.7 |
| sf | 7.43 | 92 | 1.1 | 59 | 1.7 | 38 | 2.9 | 61 | 2.1 |
| ss | 8.64 | 123 | 0.9 | 66 | 2.0 | 39 | 2.6 | 64 | 1.8 |
| no Ab |  | 27 | 0.7 | 20 | 1.0 | 30 | 1.9 | 38 | 1.0 |

(b2) CDC Activity of Humanized Antibody 1782

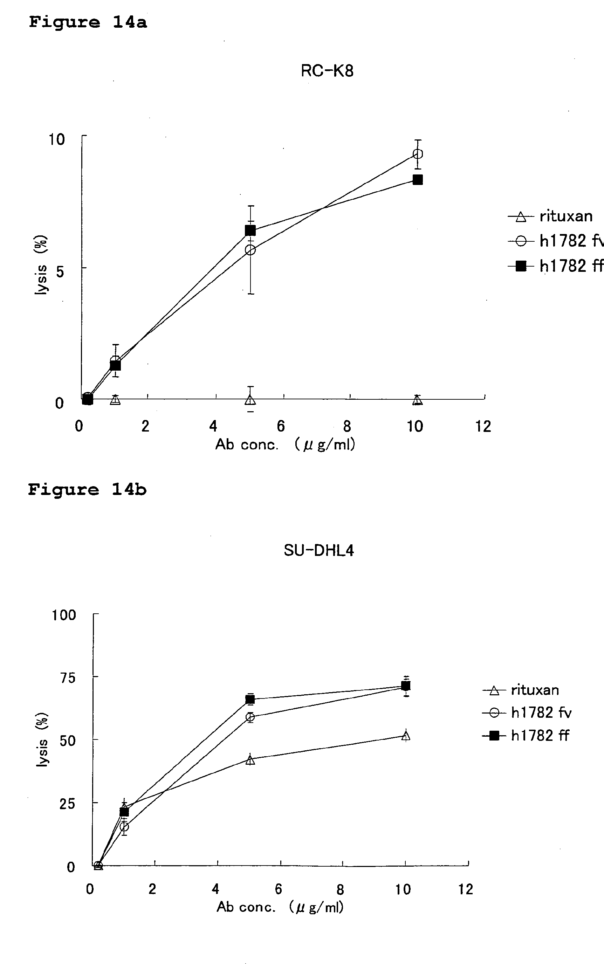

CDC activities of clones fv and ff of humanized antibody 1782 were first measured using RC-K8 and SU-DHL4 cells. The results are shown in Fig. 14a and Fig. 14b. It was found that there was not much difference between activities at antibody concentrations of 0.1 and 1 $\mu$g/ml. The clones fv and ff of humanized antibody 1782 displayed high CDC activities against either of these cell types. In the case of RC-K8 cells which are resistant to Rituxan, fv had the highest CDC activity. In the case of SU-DHL4 cells, the clones fv and ff of humanized antibody 1782 displayed high CDC activities, which were greater than that of Rituxan.
CDC activities of four clones fv, ff, sf, and ss of humanized antibody 1782 were measured using RAJI, WIL2NS, SU-DHL4, and RC-K8 cells. The results are summarized in Table 10.
CDC Activities of Humanized Antibody-1782 Clones

Table 10

| Clone | CDC (10 ug/ml) | | | |
| --- | --- | --- | --- | --- |
| | RAJI | WIL2NS | SUDHL4 | RC-K8 |
| | c2B8=100 | c2B8=100 | c2B8=100 | h1791 sf43=100 |
| c2B8 | 100 | 100 | 100 | 0 |
| ff | 94 | 68 | 138 | 19 |
| fv | 92 | 90 | 137 | 21 |
| sf | 75 | 28 | 102 | 8 |
| ss | 77 | 52 | 109 | 15 |
| h1791sf43 | | | | 100 |

These humanized antibody-1782 clones displayed CDC activities against RC-K8 cells, which are resistant to Rituxan, as well as against RAJI, WIL2NS, and SU-DHL4 cells. In particular, the clones ff and fv had high CDC activities and displayed CDC activities against SU-DHL4 cells significantly greater than that of Rituxan.
Putting together the results of Kd values and CDC activities, the clone ff is preferable in that it has the highest affinity, a relatively high CDC activity, and a CDC activity also against the strain RC-K8, a Rituxan-resistant strain, and the clone fv is preferable in that it has the highest CDC activity and in addition, is also effective against the strain RC-K8, a Rituxan-resistant strain. In particular, since the clone ff has a very high affinity, it is possible to RI label it to employ it for missile therapy of B-cell involving diseases.

(b3) ADCC Activity of Humanized Antibody 1782

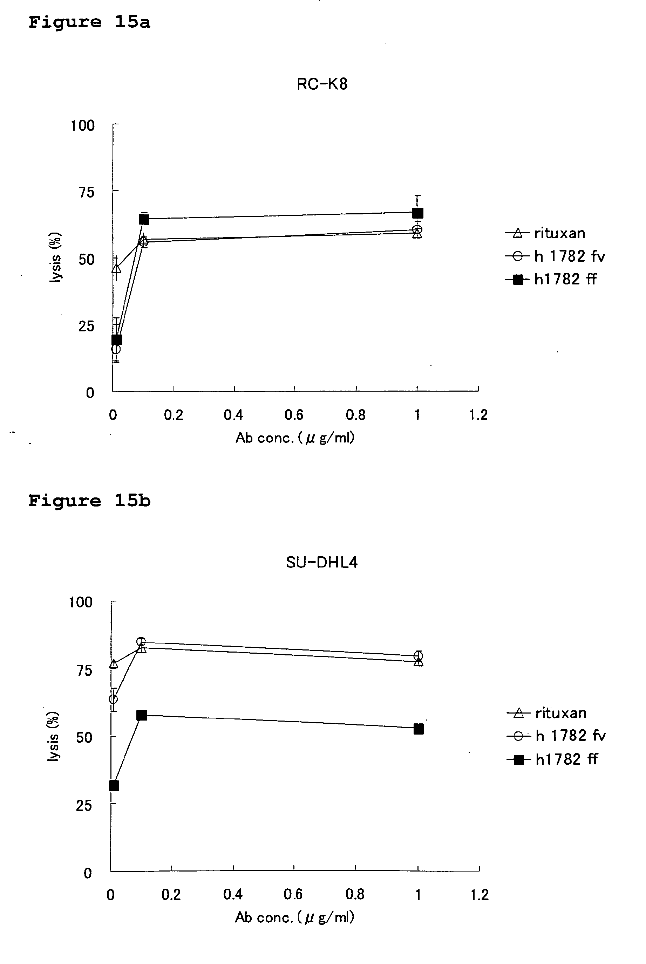

ADCC activities of the clones fv and ff of humanized antibody 1782 were first measured using RC-K8 and SU-DHL4 cells. The results are shown in Fig. 15a and Fig. 15b. It was found that there was not much difference between activities at antibody concentrations of 0.1 and 1 $\mu$g/ml.
In the case of RC-K8 cells, the clone ff had a greater ADCC activity than the clone fv and Rituxan. In the case of SU-DHL4 cells, the clone fv displayed a comparable ADCC activity to that of Rituxan, while the clone ff had a relatively low ADCC activity.
ADCC activities of the four clones fv, ff, sf, and ss of humanized antibody 1782 were measured using RAJI, WIL2NS, SU-DHL4, and RC-K8 cells. The results are summarized in Table 11.
ADCC Activities of Humanized Antibody-1782 Clones

Table 11

| Clone | ADCC (25:1 1 ug/ml) | | | |
| --- | --- | --- | --- | --- |
| | RAJI | WIL2NS | SUDHL4 | RC-K8 |
| | c2B8=100 | c2B8=100 | c2B8=100 | c2B8=100 |
| c2B8 | 100 | 100 | 100 | 100 |
| ff | 106 | 65 | 68 | 113 |

(continued)

| Clone | ADCC (25:1 1 ug/ml) | | | |
|---|---|---|---|---|
| | RAJI | WIL2NS | SUDHL4 | RC-K8 |
| | c2B8=100 | c2B8=100 | c2B8=100 | c2B8=100 |
| fv | 93 | 103 | 103 | 102 |
| sf | 109 | 99 | 70 | 144 |
| ss | 88 | 87 | 97 | 135 |

The clone fv is preferable in that it has a high ADCC activity against SU-DHL4 cell, and the clone ss is preferable in that it has a high ADCC activity against RC-K8 cells.

INDUSTRIAL APPLICABILITY

According to the present invention, there are provided anti-CD20 monoclonal antibodies, in particular, humanized antibodies, methods of producing the same, and therapeutic agents for the treatment of B-cell involving diseases containing the same, which have high binding affinities to human CD20 molecules in their natural state and display biological activities suitable as pharmaceutical use. Therefore, the present invention is applicable in the fields of manufacturing of drugs for treating cancers, cancer research, and others.

FREE TEXTS IN SEQUENCE LISTING

SEQ ID No: 17: L-chain V-region sequence of humanized antibody abb 1791
SEQ ID No: 18: L-chain V-region sequence of humanized antibody fra 1791
SEQ ID No: 19: L-chain V-region sequence of humanized antibody sdr 1791
SEQ ID No: 20: L-chain V-region sequence of humanized antibody Ven 1791
SEQ ID No: 21: H-chain V-region sequence of humanized antibody abb 1791
SEQ ID No: 22: H-chain V-region sequence of humanized antibody fra 1791
SEQ ID No: 23: H-chain V-region sequence of humanized antibody sdr 1791
SEQ ID No: 24: H-chain V-region sequence of humanized antibody Ven 1791
SEQ ID No: 25: primer
SEQ ID No: 26: primer
SEQ ID No: 27: L-chain V-region sequence of humanized antibody Ven 1782
SEQ ID No: 28: L-chain V-region sequence of humanized antibody abb 1782
SEQ ID No: 29: L-chain V-region sequence of humanized antibody sdr 1782
SEQ ID No: 30: L-chain V-region sequence of humanized antibody fra 1782
SEQ ID No: 31: H-chain V-region sequence of humanized antibody Ven 1791
SEQ ID No: 32: H-chain V-region sequence of humanized antibody abb 1782
SEQ ID No: 33: H-chain V-region sequence of humanized antibody sdr 1782
SEQ ID No: 34: H-chain V-region sequence of humanized antibody fra 1791

**Claims**

1. A humanized anti-CD20 monoclonal antibody, comprising a combination of the L-chain set forth in any one of SEQ ID Nos: 27 to 30 and the H-chain set forth in any one of SEQ ID Nos: 31 to 34.

2. The humanized anti-CD20 monoclonal antibody according to claim 1, comprising a combination of the L-chain set forth in SEQ ID No: 30 and the H-chain set forth in SEQ ID No: 34.

3. The humanized anti-CD20 monoclonal antibody according to claim 1, comprising a combination of the L-chain set forth in SEQ ID No: 30 and the H-chain set forth in SEQ ID No: 31.

4. The humanized anti-CD20 monoclonal antibody according to claim 1, comprising a combination of the L-chain set forth in SEQ ID No: 29 and the H-chain set forth in SEQ ID No: 33.

5. The humanized anti-CD20 monoclonal antibody according to claim 1, which is produced by a cell selected from the group consisting of cells which have been deposited under the accession numbers FERM ABP-10907, FERM ABP-10906, and FERM ABP-10905, with the International Patent Organism Depositary of the National Institute of Advanced Industrial Science and Technology.

6. A method for producing a humanized anti-CD20 monoclonal antibody, which comprises culturing a cell selected from the group consisting of cells which have been deposited under the accession numbers FERM ABP-10907, FERM ABP-10906, and FERM ABP-10905, with the International Patent Organism Depositary of the National Institute of Advanced Industrial Science and Technology.

7. A therapeutic agent for the treatment of B-cell mediated diseases, comprising as an active ingredient the humanized anti-CD20 monoclonal antibody according to any one of claims 1 to 6.

## Figure 1

## Figure 2

**Figure 3a**

Day1 (2ug/ml)

**Figure 3b**

Day1 (4ug/ml)

Figure 3c

Day2 (2ug/ml)

Figure 3d

Day2 (4ug/ml)

**Figure 4a**

**Figure 4b**

## Figure 4c

SU-DHL4 ADCC

Legend: rituxan, 1k0924, 1k1402, 1k1422, 1k1712, 1k1736, 1k1791

## Figure 4d

RC-K8 ADCC

Legend: rituxan, 1k0924, 1k1402, 1k1422, 1k1712, 1k1736, 1k179

## Figure 5a

Raji 5.0 μg/ml

## Figure 5b

WiL-2 5.0 μg/ml

## Figure 5c

## Figure 5d

## Figure 6a

Raji CDC

## Figure 6b

WiL2 CDC

## Figure 6c

SU-DHL4 CDC

Legend: riuxan, 1k0924, 1k1402, 1k1422, 1k1712, 1k1736, 1k1791

lysis /%

Ab conc. / μg/ml

## Figure 6d

RCK8 CDC

Legend: riuxan, 1k0924, 1k1402, 1k1422, 1k1712, 1k1736, 1k1791

lysis /%

Ab conc. / μg/ml

**Figure 7a**

**Figure 7b**

## Figure 7c

apoptosis〈SU-DHL4 mAb〉

Legend: 0.2−, 0.2+, 1−, 1+, 5−, 5+

X-axis: m2B8, m2H7, m0924, m1228, m1402, m1422, m1712, m1736, m1782, m1791, CD3, no Ab

## Figure 7d

apoptosis〈RCK8 mAb〉

Legend: 0.2−, 0.2+, 1−, 1+, 5−, 5+

X-axis: m2B8, m2H7, m0924, m1228, m1402, m1422, m1712, m1736, m1782, m1791, CD3, no Ab

## Figure 8a

## Figure 8b

## Figure 8c

apoptosis<SU-DHL4 h1791>

## Figure 8d

apoptosis<RCK8 h1791>

## Figure 9a

apoptosis⟨RAJI h1791⟩noAb=1

## Figure 9b

apoptosis⟨WIL2-NS h1791⟩ noAb=1

## Figure 9c

apoptosis⟨SU-DHL4 h1791⟩ noAb=1

## Figure 9d

apoptosis⟨RCK8 h1791⟩ noAb=1

**Figure 10a**

**Figure 10b**

## Figure 10c

## Figure 10d

Figure 11a

Ab conc. ($\mu$ g/ml)

Figure 11b

Ab conc. ($\mu$ g/ml)

**Figure 11c**

Ab conc. ($\mu$ g/ml)

Legend:
- rituxan
- c1k1791
- h1k1791 fv10
- h1k1791 ff34
- h1k1791 sf43
- h1k1791 ss32

**Figure 11d**

Ab conc. ($\mu$ g/ml)

Legend:
- rituxan
- c1k1791
- h1k1791 fv10
- h1k1791 ff34
- h1k1791 sf43
- h1k1791 ss32

## Figure 12a

**< h1782 series , RC-K8 >**

## Figure 12b

**< h1782 series , SU-DHL4 >**

## Figure 12c

< h1782 series , RAJI>

## Figure 12d

< h1782 series , WIL2NS>

## Figure 13a

< h1782 series , RC-K8 , noAb=1>

## Figure 13b

< h1782 series , SU-DHL4 , noAb=1>

## Figure 13c

< h1782 series , RAJI , noAb=1>

Legend:
- ▨ 0.2μg / ml
- ■ 0.2μg / ml ( XL )
- ▦ 1μg / ml
- ■ 1μg / ml ( XL )
- ▧ 5μg / ml
- ■ 5μg / ml ( XL )

## Figure 13d

< h1782 series , WIL2NS , noAb=1>

Legend:
- ▨ 0.2μg / ml
- ■ 0.2μg / ml ( XL )
- ▦ 1μg / ml
- ■ 1μg / ml ( XL )
- ▧ 5μg / ml
- ■ 5μg / ml ( XL )

## Figure 14a

### RC-K8

## Figure 14b

### SU-DHL4

**Figure 15a**

RC-K8

**Figure 15b**

SU-DHL4

## INTERNATIONAL SEARCH REPORT

International application No.

PCT/JP2007/067444

A. CLASSIFICATION OF SUBJECT MATTER
*C12N15/09*(2006.01)i, *A61K39/395*(2006.01)i, *A61P35/00*(2006.01)i, *A61P37/02*
(2006.01)i, *C07K16/28*(2006.01)i, *C12P21/08*(2006.01)i, *C12N5/10*(2006.01)n

According to International Patent Classification (IPC) or to both national classification and IPC

B. FIELDS SEARCHED

Minimum documentation searched (classification system followed by classification symbols)
C12N15/09, A61K39/395, A61P35/00, A61P37/02, C07K16/28, C12P21/08,
C12N5/10

Documentation searched other than minimum documentation to the extent that such documents are included in the fields searched

Electronic data base consulted during the international search (name of data base and, where practicable, search terms used)
BIOSIS/MEDLINE/WPIDS(STN), SwissProt/PIR/Geneseq, JMEDPlus(JDream2),
JST7580(JDream2), JSTPlus(JDream2)

C. DOCUMENTS CONSIDERED TO BE RELEVANT

| Category* | Citation of document, with indication, where appropriate, of the relevant passages | Relevant to claim No. |
|---|---|---|
| Y | HONG K. ET AL, Simple quantitative live cell and anti-idiotypic antibody based ELISA for humanized antibody directed to cell surface protein CD20., J.Immunol. Methods., 2004, Vol.294, No.1-2, p.189-197, full text | 1,7 |
| Y | PRESTA LG. ET AL, Humanization of an anti-vascular endothelial growth factor monoclonal antibody for the therapy of solid tumors and other disorders., Cancer Res., 1997, Vol.57, No.20, p.4593-4599, full text | 1,7 |
| Y | JP 4-502408 A (Protein Design Labs Inc.), 07 May, 1992 (07.05.92), Full text & WO 1990/007861 A1 & EP 0451216 A1 | 1,7 |

☒ Further documents are listed in the continuation of Box C.  ☐ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| | |
|---|---|
| Date of the actual completion of the international search<br>08 November, 2007 (08.11.07) | Date of mailing of the international search report<br>20 November, 2007 (20.11.07) |
| Name and mailing address of the ISA/<br>Japanese Patent Office | Authorized officer |
| Facsimile No. | Telephone No. |

Form PCT/ISA/210 (second sheet) (April 2007)

75

**INTERNATIONAL SEARCH REPORT**

| International application No. |
| --- |
| PCT/JP2007/067444 |

C (Continuation).    DOCUMENTS CONSIDERED TO BE RELEVANT

| Category* | Citation of document, with indication, where appropriate, of the relevant passages | Relevant to claim No. |
| --- | --- | --- |
| Y | Yoshiya TANAKA, "Ko CD20 Kotai", Japanese Journal of Clinical Medicine, 2005, Vol.63, special extra issue No. 5, pages 728 to 733, full text | 1,7 |

Form PCT/ISA/210 (continuation of second sheet) (April 2007)

**EP 2 186 892 A1**

| INTERNATIONAL SEARCH REPORT | International application No. |
| --- | --- |
| | PCT/JP2007/067444 |

**Box No. II     Observations where certain claims were found unsearchable (Continuation of item 2 of first sheet)**

This international search report has not been established in respect of certain claims under Article 17(2)(a) for the following reasons:

1. ☐ Claims Nos.:
   because they relate to subject matter not required to be searched by this Authority, namely:

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

**Box No. III     Observations where unity of invention is lacking (Continuation of item 3 of first sheet)**

This International Searching Authority found multiple inventions in this international application, as follows:
   (See extra sheet.)

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☒ No required additional search fees were timely paid by the applicant.   Consequently, this international search report is restricted to the invention first mentioned in the claims; it is covered by claims Nos.:
   Claims 1 and 7 (the parts depending on SEQ IDS:27 and 31).

**Remark on Protest**
the

☐ The additional search fees were accompanied by the applicant's protest and, where applicable, payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (April 2007)

**EP 2 186 892 A1**

| INTERNATIONAL SEARCH REPORT | International application No. |
|---|---|
| | PCT/JP2007/067444 |

Continuation of Box No.III of continuation of first sheet(2)

In claim 1, 16 kinds of "humanized anti-CD20 monoclonal antibodies" wherein one of L chains represented by SEQ ID NOS:27 to 30 is combined with one of H chains represented by SEQ ID NOS:31 to 34 are claimed. However, "a humanized anti-CD20 monoclonal antibody" has been publicly known (see, for example, document 1: J. Immunol. Methods, (2004), 294, [1-2], p. 189-197). Thus, it is considered that there is no "special technical feature" common to the 16 kinds of antibodies as claimed in claim 1.

There is a special technical feature, i.e., "a humanized anti-CD20 monoclonal antibody in which the L chain of SEQ ID NO:29 is combined with the H chain of SEQ ID NO:33" between claim 4 and an antibody produced by a cell having Deposition No. FERM ABP-10907; there is a special technical feature, i.e., "a humanized anti-CD20 monoclonal antibody in which the L chain of SEQ ID NO:30 is combined with the H chain of SEQ ID NO:31" between claim 3 and an antibody produced by a cell having Deposition No. FERM ABP-10906; and there is a special technical feature, i.e., "a humanized anti-CD20 monoclonal antibody in which the L chain of SEQ ID NO:30 is combined with the H chain of SEQ ID NO:34" between claim 2 and an antibody produced by a cell having Deposition No. FERM ABP-10905.

Accordingly, claims have 16 invention groups as follows.
(1)   Claims 1 and 7 (the parts depending on SEQ ID NOS:27 and 31).
(2)   Claims 1 and 7 (the parts depending on SEQ ID NOS:27 and 32).
(3)   Claims 1 and 7 (the parts depending on SEQ ID NOS:27 and 33).
(4)   Claims 1 and 7 (the parts depending on SEQ ID NOS:27 and 34).
(5)   Claims 1 and 7 (the parts depending on SEQ ID NOS:28 and 31).
(6)   Claims 1 and 7 (the parts depending on SEQ ID NOS:28 and 32).
(7)   Claims 1 and 7 (the parts depending on SEQ ID NOS:28 and 33).
(8)   Claims 1 and 7 (the parts depending on SEQ ID NOS:28 and 34).
(9)   Claims 1 and 7 (the parts depending on SEQ ID NOS:29 and 31).
(10)  Claims 1 and 7 (the parts depending on SEQ ID NOS:29 and 32).
(11)  Claims 1 and 7 (the parts depending on SEQ ID NOS:29 and 33) and claim 4 and claims 5 and 6 (the parts depending on FERM ABP-10907).
(12)  Claims 1 and 7 (the parts depending on SEQ ID NOS:29 and 34).
(13)  Claims 1 and 7 (the parts depending on SEQ ID NOS:30 and 31) and claim 3 and claims 5 and 6 (the parts depending on FERM ABP-10906).
(14)  Claims 1 and 7 (the parts depending on SEQ ID NOS:30 and 32).
(15)  Claims 1 and 7 (the parts depending on SEQ ID NOS:30 and 33).
(16)  Claims 1 and 7 (the parts depending on SEQ ID NOS:30 and 34) and claim 2 and claims 5 and 6 (the parts depending on FERM ABP-10905).

Form PCT/ISA/210 (extra sheet) (April 2007)

78

**EP 2 186 892 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 9411026 A
- US 5736137 A

- JP 3582965 B

### Non-patent literature cited in the description

- **Coiffier B et al.** *Blood,* 1998, vol. 92, 1297-32
- **Edward JC et al.** *Rheumatology (Oxford),* 2001, vol. 40, 205-11
- **Zaja F et al.** *Heamatologica,* 2002, vol. 87, 189-95
- **Perrotta S et al.** *Br J Haematol,* 2002, vol. 116, 465-7
- **Reff et al.** *Blood,* 1994, vol. 83, 435-445
- **Maloney et al.** *Blood,* 1996, vol. 88, 637a
- *IDEC Pharmaceuticals Corporation News Release,* 08 December 1998
- **Mitchell ER et al.** *Blood,* 1994, vol. 82, 435-445
- **Miyamoto T ; Min W ; Lillehoj HS.** *Avian Dis.,* January 2002, vol. 46 (1), 10-6

- **Ishida T ; Imai K.** *Nippon Rinsho,* March 2002, vol. 60 (3), 439-444
- **Eduardo A. Padlan.** *Molecular Immunology,* 1991, vol. 28-4/5, 489-498
- **Eduardo A. Padlan.** *The FASEB Journal,* vol. 9, 133-139
- **Tai te Wu ; Elvin A. Kabat.** *Molecular Immunology,* 1992, vol. 29 (9), 1141-1146
- **Monokuronaru kotai ; Seikagaku Jikkenho.** Monoclonal Antibody, Biochemical Experiment Methods. 1989
- **Scatchard, G.** *Ann. N.Y. Acad. Sci.,* 1949, vol. 51, 660-672
- New Cultured Cell Experimental Methods in Molecular Biology Research. Yodosha Co., Ltd, 212-217

[SEQUENCE LISTING]

SEQUENCE LISTING

<110> OSAKA UNIVERSITY

<120> Anti CD20 monoclonal antibody

<130> 667816

<160> 34

<170> PatentIn version 3.2

<210> 1
<211> 106
<212> PRT
<213> Mus musculus

<400> 1

Gln Ile Val Leu Thr Gln Ser Pro Pro Ile Met Ser Ala Ser Leu Gly
1               5                   10                  15

Glu Glu Ile Thr Leu Thr Cys Ser Ala Ser Ser Arg Val Ser Tyr Met
            20                  25                  30

Leu Trp Tyr Gln Gln Lys Ser Gly Thr Ser Pro Lys Leu Leu Ile Tyr
        35                  40                  45

Ser Thr Ser Asn Leu Ala Ser Gly Val Pro Ser Arg Phe Ser Gly Ser
    50                  55                  60

Gly Ser Gly Thr Phe Tyr Ser Leu Thr Ile Ser Ser Val Glu Ala Glu
65                  70                  75                  80

Asp Ala Ala Asp Tyr Tyr Cys His Gln Trp Thr Ser Asn Pro Cys Thr
                85                  90                  95

Phe Gly Gly Gly Thr Lys Leu Glu Ile Lys
            100                 105

<210> 2
<211> 107
<212> PRT
<213> Mus musculus

<400> 2

Ser Thr Val Met Thr Gln Thr Pro Lys Phe Leu Leu Val Ser Ala Gly
1               5                   10                  15

Asp Arg Val Thr Ile Thr Cys Lys Ala Ser Gln Ser Val Ser Asn Asp
            20                  25                  30

Val Ala Trp Tyr Gln Gln Lys Pro Gly Gln Ser Pro Lys Val Leu Ile

                 35                  40                  45

```
Tyr Phe Ala Ser Asn Arg Tyr Thr Gly Val Pro Asp Arg Phe Thr Gly
    50                  55                  60

Ser Gly Tyr Gly Thr Asp Phe Thr Phe Thr Ile Asn Thr Val Gln Ala
65                  70                  75                  80

Glu Asp Leu Ala Val Tyr Phe Cys Gln Gln Asp Tyr Ser Ser Pro Leu
                85                  90                  95

Thr Phe Gly Ala Gly Thr Lys Leu Glu Leu Lys
            100                 105
```

<210> 3
<211> 106
<212> PRT
<213> Mus musculus

<400> 3

```
Gln Ile Val Leu Ser Gln Ser Pro Ala Ile Leu Ser Ala Ser Pro Gly
1               5                   10                  15

Glu Lys Val Thr Met Thr Cys Arg Ala Ser Ser Ser Val Ser Tyr Met
            20                  25                  30

His Trp Tyr Gln Gln Arg Pro Gly Ser Ser Pro Lys Pro Trp Ile Tyr
            35                  40                  45

Ala Thr Ser Asn Leu Ala Ser Gly Val Pro Ala Arg Phe Ser Gly Ser
    50                  55                  60

Gly Ser Gly Thr Ser Tyr Tyr Phe Thr Ile Ser Arg Val Glu Ala Glu
65                  70                  75                  80

Asp Ala Ala Thr Tyr Tyr Cys Gln Gln Trp Asn Ser Asn Pro Pro Thr
                85                  90                  95

His Gly Gly Gly Thr Lys Leu Glu Ile Lys
            100                 105
```

<210> 4
<211> 106
<212> PRT
<213> Mus musculus

<400> 4

```
Gln Ile Val Leu Ser Gln Ser Pro Ala Ile Leu Ser Ala Ser Pro Gly
1               5                   10                  15
```

Glu Lys Val Thr Met Thr Cys Arg Ala Ser Ser Ser Val Ser Tyr Met
                20                  25                  30

Asp Trp Tyr Gln Gln Lys Pro Gly Ser Ser Pro Lys Pro Trp Ile Tyr
            35                  40                  45

Ala Thr Ser Asn Leu Ala Ser Gly Val Pro Ala Arg Phe Ser Gly Ser
        50                  55                  60

Gly Ser Gly Thr Ser Tyr Ser Leu Thr Ile Ser Arg Val Glu Ala Glu
65                  70                  75                  80

Asp Thr Ala Thr Tyr Tyr Cys Gln Gln Trp Thr Phe Asn Pro Pro Thr
                85                  90                  95

Phe Gly Ser Gly Thr Lys Leu Glu Ile Lys
                100                 105


<210>   5
<211>   106
<212>   PRT
<213>   Mus musculus

<400>   5

Gln Ile Val Leu Ser Gln Ser Pro Ala Ile Leu Ser Ala Ser Pro Gly
1                   5                   10                  15

Glu Lys Val Thr Met Thr Cys Arg Ala Ser Ser Ser Val Ser Tyr Met
                20                  25                  30

His Trp Tyr Gln Gln Lys Pro Gly Ser Ser Pro Lys Pro Trp Ile Tyr
            35                  40                  45

Ala Thr Ser Asn Leu Ala Ser Gly Val Pro Ala Arg Phe Ser Gly Ser
        50                  55                  60

Gly Ser Gly Thr Ser Tyr Ser Leu Thr Ile Thr Arg Val Glu Ala Glu
65                  70                  75                  80

Asp Ala Ala Thr Tyr Tyr Cys Gln Gln Trp Thr Phe Asn Pro Pro Thr
                85                  90                  95

Phe Gly Ala Gly Thr Lys Leu Glu Leu Lys
                100                 105


<210>   6
<211>   106

```
<212>   PRT
<213>   Mus musculus

<400>   6

Gln Ile Val Leu Ser Gln Ser Pro Ala Ile Leu Ser Ser Ser Pro Gly
1                   5                   10                  15

Glu Lys Val Thr Met Thr Cys Arg Ala Ser Ser Ser Val Ser Tyr Met
            20                  25                  30

Leu Trp Tyr Gln Gln Lys Pro Gly Ser Ser Pro Glu Pro Trp Ile Tyr
        35                  40                  45

Ala Thr Ser Asn Leu Ala Ser Gly Val Pro Ala Arg Phe Ser Gly Gly
    50                  55                  60

Gly Ser Gly Thr Ser Tyr Ser Leu Thr Ile Ser Arg Val Glu Ala Glu
65                  70                  75                  80

Asp Ala Ala Thr Tyr Tyr Cys Gln Gln Trp Thr Phe Asn Pro Pro Thr
                85                  90                  95

Phe Gly Gly Gly Thr Lys Leu Glu Ile Lys
            100                 105


<210>   7
<211>   107
<212>   PRT
<213>   Mus musculus

<400>   7

Asp Ile Leu Leu Thr Gln Ser Pro Ala Ile Leu Phe Val Ser Pro Gly
1                   5                   10                  15

Glu Arg Val Ser Leu Ser Cys Arg Ala Ser Gln Asn Ile Gly Thr Ser
            20                  25                  30

Ile His Trp Tyr Gln Gln Arg Thr Asn Gly Ser Pro Arg Leu Leu Ile
        35                  40                  45

Lys Tyr Ala Ser Glu Ser Phe Ser Gly Ile Pro Ser Arg Phe Ser Gly
    50                  55                  60

Ser Gly Ser Gly Thr Asp Phe Thr Leu Ser Ile Asn Ser Val Glu Ser
65                  70                  75                  80

Glu Asp Ile Ala Asp Tyr Tyr Cys Gln Gln Ser Asn Ser Trp Pro Phe
                85                  90                  95

Thr Phe Gly Ser Gly Thr Lys Leu Glu Ile Lys
            100                 105


<210>   8
```

```
<211>  108
<212>  PRT
<213>  Mus musculus

<400>  8

Glu Ile Ile Leu Thr Gln Ser Pro Thr Thr Met Ala Ala Ser Pro Gly
1               5                   10                  15

Glu Lys Ile Thr Ile Thr Cys Ser Ala Ser Ser Ser Ile Ser Ser Tyr
            20                  25                  30

Tyr Leu Arg Trp Tyr Gln Gln Lys Pro Gly Phe Ser Pro Lys Val Leu
            35                  40                  45

Ile Tyr Arg Thr Ser Asn Leu Ala Ser Gly Val Pro Ala Arg Phe Ser
        50                  55                  60

Gly Ser Gly Ser Gly Thr Ser Tyr Ser Leu Thr Ile Gly Thr Met Glu
65                  70                  75                  80

Ala Glu Asp Val Ala Thr Tyr Tyr Cys Gln Gln Gly Asn Thr Val Pro
                85                  90                  95

Leu Thr Phe Gly Ser Gly Thr Lys Leu Glu Ile Lys
                100                 105


<210>  9
<211>  123
<212>  PRT
<213>  Mus musculus

<400>  9

Gln Val Gln Leu Gln Gln Pro Gly Ala Glu Leu Val Lys Pro Gly Ala
1               5                   10                  15

Ser Val Lys Met Ser Cys Arg Ala Ser Gly Tyr Thr Phe Thr Asn Tyr
            20                  25                  30

Asn Met His Trp Ile Lys Gln Thr Pro Gly Gln Gly Leu Glu Trp Ile
            35                  40                  45

Gly Ala Ile Tyr Pro Gly Ser Gly Asp Thr Ser Tyr Asn Arg Lys Phe
        50                  55                  60

Lys Gly Lys Ala Thr Leu Thr Ala Asp Thr Ser Ser Ser Thr Ala Tyr
65                  70                  75                  80

Met Gln Phe Ser Ser Leu Thr Ser Ala Asp Ser Ala Val Tyr Tyr Cys
                85                  90                  95
```

Ala Arg Phe Thr Tyr Tyr Tyr Gly Gly Thr Tyr Gly Ala Met Asp Tyr
            100                     105                 110

Trp Gly Gln Gly Thr Ser Val Thr Val Ser Leu
            115                 120

<210> 10
<211> 124
<212> PRT
<213> Mus musculus

<400> 10

Gln Ile Gln Leu Val Gln Ser Gly Pro Glu Leu Lys Lys Pro Gly Glu
1                   5                   10                  15

Thr Val Lys Ile Ser Cys Lys Ala Ser Gly Tyr Thr Phe Thr Asn Phe
            20                  25                  30

Gly Val Asn Trp Val Lys Gln Ala Pro Gly Lys Gly Leu Lys Trp Met
            35                  40                  45

Gly Trp Ile Asn Thr Tyr Thr Gly Glu Pro Ser Tyr Ala Asp Asp Phe
        50                  55                  60

Lys Gly Arg Phe Ala Phe Ser Leu Glu Ala Ser Ala Asn Thr Ala Tyr
65                  70                  75                  80

Leu Gln Ile Asn Asn Leu Lys Asn Asp Asp Met Ser Thr Tyr Phe Cys
                85                  90                  95

Thr Arg Arg Thr Asn Tyr Tyr Gly Thr Ser Tyr Tyr Tyr Ala Met Asp
            100                     105                 110

Tyr Trp Gly Gln Gly Thr Ser Val Thr Val Ser Ser
            115                 120

<210> 11
<211> 119
<212> PRT
<213> Mus musculus

<400> 11

Gln Val Gln Leu Gln Gln Pro Gly Ala Glu Leu Val Lys Pro Gly Ala
1                   5                   10                  15

Ser Val Lys Met Ser Cys Lys Ala Ser Gly Tyr Thr Phe Thr Ser Tyr
            20                  25                  30

Asn Ile His Trp Val Lys Gln Thr Pro Gly Gln Gly Leu Glu Trp Ile
35                    40                   45

Gly Ala Ile Tyr Pro Gly Asn Gly Asp Thr Ser Tyr Asn Gln Lys Phe
50                   55                 60

Lys Gly Lys Ala Thr Leu Thr Ser Asp Lys Ser Ser Ser Thr Ala Tyr
65                  70                 75                      80

Met Gln Leu Ser Ser Leu Thr Ser Glu Asp Ser Ala Val Tyr Tyr Cys
85                          90                   95

Ala Arg Met Ser Thr Met Ile Thr Gly Phe Asp Tyr Trp Gly Gln Gly
100                    105                   110

Thr Thr Leu Thr Val Ser Ser
115

<210> 12
<211> 122
<212> PRT
<213> Mus musculus

<400> 12

Gln Val Gln Leu Gln Gln Pro Gly Ala Glu Leu Val Lys Pro Gly Ala
1               5                    10                  15

Ser Val Lys Met Ser Cys Lys Ala Ser Gly Phe Thr Phe Thr Ser Tyr
20                  25                 30

Asn Leu His Trp Val Lys Gln Thr Pro Gly Gln Gly Leu Glu Trp Ile
35                    40                   45

Gly Ala Ile Tyr Pro Gly Ser Gly Asp Thr Ser Tyr Asn Gln Gln Phe
50                   55                 60

Lys Gly Lys Ala Thr Leu Thr Ala Asp Lys Ser Ser Asn Thr Ala Tyr
65                  70                 75                      80

Met Gln Leu Asn Ser Leu Thr Ser Glu Asp Ser Ala Val Tyr Cys Cys
85                          90                   95

Ala Arg Ser Ala Met Ile Ser Thr Gly Asn Trp Tyr Phe Asp Tyr Trp
100                    105                   110

Gly Gln Gly Thr Thr Leu Thr Val Ser Ser
115                      120

<210> 13
<211> 121
<212> PRT
<213> Mus musculus

<400> 13

Gln Val Gln Leu Gln Gln Pro Gly Ala Glu Leu Val Lys Pro Gly Ala
1               5                   10                  15

Ser Val Lys Met Ser Cys Lys Ala Ser Gly Phe Thr Phe Thr Ser Tyr
                20                  25                  30

Asn Met His Trp Val Lys Gln Thr Pro Gly Gln Gly Leu Glu Trp Ile
            35                  40                  45

Gly Gly Ile Tyr Pro Gly Asn Gly Asp Thr Ser Tyr Asn Gln Lys Phe
        50                  55                  60

Lys Gly Lys Ala Thr Leu Thr Ala Asp Lys Ser Ser Ser Thr Ala Tyr
65                  70                  75                  80

Met Gln Leu Ser Ser Leu Thr Ser Glu Asp Ser Ala Val Tyr Tyr Cys
                85                  90                  95

Ala Arg Phe Tyr Tyr Tyr Gly Ser Met Gly Ala Met Asp Tyr Trp Gly
            100                 105                 110

Gln Gly Thr Ser Val Thr Val Ser Ser
            115                 120

<210> 14
<211> 122
<212> PRT
<213> Mus musculus

<400> 14

Gln Val Gln Leu Gln Gln Pro Gly Ala Glu Leu Val Lys Pro Gly Ala
1               5                   10                  15

Ser Val Lys Met Ser Cys Lys Ala Ser Gly Tyr Thr Phe Thr Thr Tyr
                20                  25                  30

Asn Leu His Trp Val Lys Gln Thr Pro Gly Gln Gly Leu Glu Trp Ile
            35                  40                  45

Gly Gly Ile Tyr Pro Gly Asn Gly Asp Thr Ser Tyr Asn Gln Lys Phe
        50                  55                  60

Lys Val Lys Ala Thr Leu Thr Ala Asp Lys Ser Ser Asn Thr Ala Tyr
65              70              75                      80

Met Gln Leu Ser Ser Leu Thr Ser Glu Asp Ser Ala Val Tyr Tyr Cys
                85                  90                  95

Ala Arg Trp Ile Tyr Tyr Gly Asn Tyr Glu Gly Thr Leu Asp Tyr Trp
            100                 105                 110

Gly Gln Gly Thr Ser Val Thr Val Ser Ser
        115                 120

<210>   15
<211>   116
<212>   PRT
<213>   Mus musculus

<400>   15

Gln Val Gln Leu Gln Gln Ser Gly Ala Glu Leu Ala Lys Pro Gly Ala
1                   5                   10                  15

Ser Val Lys Met Ser Cys Lys Ala Ser Gly Tyr Thr Phe Thr Ser Tyr
                20                  25                  30

Trp Met His Trp Val Lys Gln Arg Pro Gly Gln Gly Leu Glu Trp Ile
            35                  40                  45

Gly Tyr Ile Thr Pro Ser Thr Gly Tyr Thr Asp Tyr Asn Lys Lys Phe
        50                  55                  60

Lys Asp Lys Ala Thr Leu Thr Ala Asp Arg Ser Ser Ser Thr Ala Tyr
65              70              75                      80

Met His Leu Ser Ser Leu Thr Ser Glu Asp Ser Ala Val Tyr Tyr Cys
                85                  90                  95

Ala Arg Ser Gly Pro Tyr Phe Asp Val Trp Gly Ala Gly Thr Thr Val
            100                 105                 110

Thr Val Ser Ser
        115

<210>   16
<211>   119
<212>   PRT
<213>   Mus musculus

<400>   16

Gln Val Gln Leu Gln Gln Pro Gly Ala Glu Leu Val Lys Pro Gly Ala

```
          1                  5                  10                 15

          Ser Val Lys Val Ser Cys Lys Ala Ser Gly Phe Thr Phe Thr Ser Tyr
                      20              25                  30

          Asn Leu His Trp Val Lys Gln Thr Pro Gly Gln Gly Leu Val Trp Ile
                      35              40                  45

          Gly Ala Ile Tyr Pro Gly Asn Gly Asp Thr Ser Tyr Asn Gln Lys Phe
                  50              55              60

          Arg Gly Lys Ala Thr Leu Thr Ala Asp Ile Ser Ser Ser Thr Ala Tyr
          65                  70              75                  80

          Met Gln Leu Ser Ser Leu Thr Ser Glu Asp Ser Ala Val Tyr Tyr Cys
                          85              90                  95

          Ala Arg Tyr Tyr Tyr Gly Tyr Asp Ala Met Asp Tyr Trp Gly Gln Gly
                      100             105                 110

          Thr Ser Val Thr Val Ser Ser
                      115


          <210>  17
          <211>  107
          <212>  PRT
          <213>  Artificial

          <220>
          <223>  L chain V region sequence of humanized antibody abb 1791

          <400>  17

          Ser Thr Val Met Thr Gln Ser Pro Asp Ser Leu Ala Val Ser Leu Gly
          1                   5                   10                  15

          Glu Arg Ala Thr Ile Asn Cys Lys Ser Ser Gln Ser Val Ser Asn Asp
                      20              25                  30

          Val Ala Trp Tyr Gln Gln Lys Pro Gly Gln Ser Pro Lys Val Leu Ile
                      35              40                  45

          Tyr Phe Ala Ser Asn Arg Tyr Ser Gly Val Pro Asp Arg Phe Ser Gly
                  50              55              60

          Ser Gly Tyr Gly Thr Asp Phe Thr Leu Thr Ile Ser Ser Leu Gln Ala
          65                  70              75                  80

          Glu Asp Val Ala Val Tyr Phe Cys Gln Gln Asp Tyr Ser Ser Pro Leu
                          85              90                  95
```

Thr Phe Gly Ala Gly Thr Lys Leu Glu Ile Lys
            100                 105


<210> 18
<211> 107
<212> PRT
<213> Artificial

<220>
<223> L chain V region sequence of humanized antibody fra 1791

<400> 18

Ser Thr Val Met Thr Gln Ser Pro Ser Phe Leu Ser Ala Ser Val Gly
1               5               10              15

Asp Arg Val Thr Ile Thr Cys Lys Ala Ser Gln Ser Val Ser Asn Asp
            20              25              30

Val Ala Trp Tyr Gln Gln Lys Pro Gly Gln Ser Pro Lys Val Leu Ile
            35              40              45

Tyr Phe Ala Ser Asn Arg Tyr Thr Gly Val Pro Asp Arg Phe Ser Gly
    50              55              60

Ser Gly Tyr Gly Thr Asp Phe Thr Leu Thr Ile Ser Ser Leu Gln Ala
65              70              75              80

Glu Asp Val Ala Val Tyr Phe Cys Gln Gln Asp Tyr Ser Ser Pro Leu
            85              90              95

Thr Phe Gly Ala Gly Thr Lys Leu Glu Ile Lys
            100                 105


<210> 19
<211> 107
<212> PRT
<213> Artificial

<220>
<223> L chain V region sequence of humanized antibody sdr 1791

<400> 19

Ser Thr Val Met Thr Gln Ser Pro Asp Ser Leu Ala Val Ser Leu Gly
1               5               10              15

Glu Arg Ala Thr Ile Asn Cys Lys Ser Ser Gln Ser Asn Ser Asn Asp
            20              25              30

Val Ala Trp Tyr Gln Gln Lys Pro Gly Gln Ser Pro Lys Val Leu Ile

35                    40                    45

Tyr Phe Ala Ser Asn Arg Tyr Ser Gly Val Pro Asp Arg Phe Ser Gly
        50                  55                  60

Ser Gly Tyr Gly Thr Asp Phe Thr Leu Thr Ile Ser Ser Leu Gln Ala
65                  70                  75                  80

Glu Asp Val Ala Val Tyr Phe Cys Gln Gln Asp Tyr Ser Ser Pro Leu
                85                  90                  95

Thr Phe Gly Ala Gly Thr Lys Leu Glu Ile Lys
            100                 105

<210> 20
<211> 107
<212> PRT
<213> Artificial

<220>
<223> L chain V region sequence of humanized antibody Ven 1791

<400> 20

Ser Thr Val Met Thr Gln Ser Pro Asp Ser Leu Ala Val Ser Leu Gly
1                   5                   10                  15

Glu Arg Val Thr Ile Asn Cys Lys Ala Ser Gln Ser Val Ser Asn Asp
            20                  25                  30

Val Ala Trp Tyr Gln Gln Lys Pro Gly Gln Ser Pro Lys Val Leu Ile
            35                  40                  45

Tyr Phe Ala Ser Asn Arg Tyr Thr Gly Val Pro Asp Arg Phe Ser Gly
        50                  55                  60

Ser Gly Tyr Gly Thr Asp Phe Thr Phe Thr Ile Ser Ser Val Gln Ala
65                  70                  75                  80

Glu Asp Val Ala Val Tyr Phe Cys Gln Gln Asp Tyr Ser Ser Pro Leu
                85                  90                  95

Thr Phe Gly Ala Gly Thr Lys Leu Glu Leu Lys
            100                 105

<210> 21
<211> 124
<212> PRT
<213> Artificial

<220>

<223> H chain V region sequence of humanized antibody abb 1791

<400> 21

Gln Ile Gln Leu Val Gln Ser Gly Ser Glu Leu Lys Lys Pro Gly Ala
1               5                   10                  15

Ser Val Lys Val Ser Cys Lys Ala Ser Gly Tyr Thr Phe Thr Asn Phe
            20                  25                  30

Gly Val Asn Trp Val Arg Gln Ala Pro Gly Lys Gly Leu Glu Trp Met
            35                  40                  45

Gly Trp Ile Asn Thr Tyr Thr Gly Glu Pro Ser Tyr Ala Gln Gly Phe
        50                  55                  60

Thr Gly Arg Phe Val Phe Ser Leu Asp Ala Ser Val Ser Thr Ala Tyr
65                  70                  75                  80

Leu Gln Ile Ser Ser Leu Lys Ala Glu Asp Thr Ala Thr Tyr Phe Cys
                85                  90                  95

Thr Arg Arg Thr Asn Tyr Tyr Gly Thr Ser Tyr Tyr Tyr Ala Met Asp
            100                 105                 110

Tyr Trp Gly Gln Gly Thr Thr Val Thr Val Ser Ser
            115                 120


<210> 22
<211> 124
<212> PRT
<213> Artificial

<220>
<223> H chain V region sequence of humanized antibody fra 1791

<400> 22

Gln Ile Gln Leu Val Gln Ser Gly Ser Glu Leu Lys Lys Pro Gly Ala
1               5                   10                  15

Ser Val Lys Val Ser Cys Lys Ala Ser Gly Tyr Thr Phe Thr Asn Phe
            20                  25                  30

Gly Val Asn Trp Val Lys Gln Ala Pro Gly Lys Gly Leu Lys Trp Met
            35                  40                  45

Gly Trp Ile Asn Thr Tyr Thr Gly Glu Pro Ser Tyr Ala Asp Asp Phe
        50                  55                  60

Lys Gly Arg Phe Ala Phe Ser Leu Asp Ala Ser Ala Ser Thr Ala Tyr

                65                        70                        75                        80

Leu Gln Ile Ser Ser Leu Lys Ala Glu Asp Met Ala Thr Tyr Phe Cys
                    85                        90                        95

Thr Arg Arg Thr Asn Tyr Tyr Gly Thr Ser Tyr Tyr Tyr Ala Met Asp
                100                        105                        110

Tyr Trp Gly Gln Gly Thr Thr Val Thr Val Ser Ser
                115                        120

<210> 23
<211> 124
<212> PRT
<213> Artificial

<220>
<223> H chain V region sequence of humanized antibody sdr 1791

<400> 23

Gln Ile Gln Leu Val Gln Ser Gly Ser Glu Leu Lys Lys Pro Gly Ala
1                   5                        10                        15

Ser Val Lys Val Ser Cys Lys Ala Ser Gly Tyr Thr Phe Thr Asn Phe
                20                        25                        30

Gly Val Asn Trp Val Arg Gln Ala Pro Gly Lys Gly Leu Lys Trp Met
                35                        40                        45

Gly Trp Ile Asn Thr Tyr Thr Gly Glu Pro Ser Tyr Ala Gln Gly Phe
                50                        55                        60

Thr Gly Arg Phe Ala Phe Ser Leu Asp Ala Ser Val Ser Thr Ala Tyr
65                        70                        75                        80

Leu Gln Ile Ser Ser Leu Lys Ala Glu Asp Thr Ala Thr Tyr Phe Cys
                    85                        90                        95

Thr Arg Arg Thr Asn Tyr Tyr Gly Thr Ser Tyr Tyr Tyr Ala Met Asp
                100                        105                        110

Tyr Trp Gly Gln Gly Thr Thr Val Thr Val Ser Ser
                115                        120

<210> 24
<211> 124
<212> PRT
<213> Artificial

<220>

<223> H chain V region sequence of humanized antibody Ven 1791

<400> 24

```
Gln Ile Gln Leu Val Gln Ser Gly Pro Glu Leu Lys Lys Pro Gly Ala
1               5              10                  15

Ser Val Lys Ile Ser Cys Lys Ala Ser Gly Tyr Thr Phe Thr Asn Phe
            20              25              30

Gly Val Asn Trp Val Lys Gln Ala Pro Gly Lys Gly Leu Lys Trp Met
        35              40              45

Gly Trp Ile Asn Thr Tyr Thr Gly Glu Pro Ser Tyr Ala Asp Asp Phe
    50              55              60

Lys Gly Arg Phe Ala Phe Ser Leu Asp Ala Ser Val Ser Thr Ala Tyr
65              70              75              80

Leu Gln Ile Ser Ser Leu Lys Ala Glu Asp Thr Ser Thr Tyr Phe Cys
                85              90              95

Thr Arg Arg Thr Asn Tyr Tyr Gly Thr Ser Tyr Tyr Tyr Ala Met Asp
            100             105             110

Tyr Trp Gly Gln Gly Thr Thr Val Thr Val Ser Ser
        115             120
```

<210> 25
<211> 35
<212> DNA
<213> Artificial sequence

<220>
<223> primer

<400> 25

aatgcggccg ccaccatgac aacacccaga aattc                    35

<210> 26
<211> 29
<212> DNA
<213> Artificial sequence

<220>
<223> primer

<400> 26

gctctagatt aaggagagct gtcattttc                           29

<210> 27

<211> 107
<212> PRT
<213> Artificial

<220>
<223> L chain V region sequence of humanized antibody Ven 1782

<400> 27

Asp Ile Leu Leu Thr Gln Ser Pro Ala Thr Leu Ser Leu Ser Pro Gly
1               5                   10                  15

Glu Arg Ala Thr Leu Ser Cys Arg Ala Ser Gln Asn Ile Gly Thr Ser
            20                  25                  30

Ile His Trp Tyr Gln Gln Lys Pro Gly Gln Ser Pro Arg Leu Leu Ile
        35                  40                  45

Lys Tyr Ala Ser Glu Ser Phe Ser Gly Ile Pro Ser Arg Phe Ser Gly
    50                  55                  60

Ser Gly Ser Gly Thr Asp Phe Thr Leu Thr Ile Ser Ser Leu Glu Pro
65                  70                  75                  80

Glu Asp Phe Ala Asp Tyr Tyr Cys Gln Gln Ser Asn Ser Trp Pro Phe
                85                  90                  95

Thr Phe Gly Ser Gly Thr Lys Leu Glu Ile Lys
            100                 105

<210> 28
<211> 107
<212> PRT
<213> Artificial

<220>
<223> L chain V region sequence of humanized antibody abb 1782

<400> 28

Asp Ile Val Leu Thr Gln Ser Pro Ala Thr Leu Ser Leu Ser Pro Gly
1               5                   10                  15

Glu Arg Ala Thr Leu Ser Cys Arg Ala Ser Gln Asn Ile Gly Thr Ser
            20                  25                  30

Ile His Trp Tyr Gln Gln Lys Pro Gly Gln Ser Pro Arg Leu Leu Ile
        35                  40                  45

Lys Tyr Ala Ser Glu Ser Phe Thr Gly Ile Pro Ser Arg Phe Ser Gly
    50                  55                  60

```
Ser Gly Ser Gly Thr Asp Phe Thr Leu Thr Ile Ser Ser Leu Glu Pro
65                  70                  75                  80

Glu Asp Phe Ala Asp Tyr Tyr Cys Gln Gln Ser Asn Ser Trp Pro Phe
                85                  90                  95

Thr Phe Gly Ser Gly Thr Lys Leu Glu Ile Lys
            100                 105
```

```
<210>  29
<211>  107
<212>  PRT
<213>  Artificial

<220>
<223>  L chain V region sequence of humanized antibody sdr 1782

<400>  29
```

```
Asp Ile Leu Leu Thr Gln Ser Pro Ala Thr Leu Ser Leu Ser Pro Gly
1               5                   10                  15

Glu Arg Ala Thr Leu Ser Cys Arg Ala Ser Gln Asn Val Gly Thr Ser
            20                  25                  30

Leu His Trp Tyr Gln Gln Lys Pro Gly Gln Ser Pro Arg Leu Leu Ile
        35                  40                  45

Lys Tyr Ala Ser Glu Arg Phe Thr Gly Ile Pro Ser Arg Phe Ser Gly
    50                  55                  60

Ser Gly Ser Gly Thr Asp Phe Thr Leu Thr Ile Ser Ser Leu Glu Pro
65                  70                  75                  80

Glu Asp Phe Ala Asp Tyr Tyr Cys Gln Gln Ser Asn Ser Trp Pro Phe
                85                  90                  95

Thr Phe Gly Ser Gly Thr Lys Leu Glu Ile Lys
            100                 105
```

```
<210>  30
<211>  107
<212>  PRT
<213>  Artificial

<220>
<223>  L chain V region sequence of humanized antibody fra 1782

<400>  30
```

```
Asp Ile Leu Leu Thr Gln Ser Pro Ala Thr Leu Ser Val Ser Pro Gly
1               5                   10                  15
```

Glu Arg Ala Thr Leu Ser Cys Arg Ala Ser Gln Asn Ile Gly Thr Ser
             20                  25                  30

Ile His Trp Tyr Gln Gln Arg Pro Gly Gln Ser Pro Arg Leu Leu Ile
         35                  40                  45

Lys Tyr Ala Ser Glu Ser Phe Ser Gly Ile Pro Ser Arg Phe Ser Gly
     50                  55                  60

Ser Gly Ser Gly Thr Asp Phe Thr Leu Thr Ile Asn Ser Leu Gln Pro
65               70                  75                  80

Glu Asp Ile Ala Asp Tyr Tyr Cys Gln Gln Ser Asn Ser Trp Pro Phe
             85                  90                  95

Thr Phe Gly Ser Gly Thr Lys Leu Glu Ile Lys
             100                 105

<210> 31
<211> 116
<212> PRT
<213> Artificial

<220>
<223> H chain V region sequence of humanized antibody Ven 1782

<400> 31

Gln Val Gln Leu Val Gln Ser Gly Ala Glu Leu Lys Lys Pro Gly Ala
1               5                   10                  15

Ser Val Lys Val Ser Cys Lys Ala Ser Gly Tyr Thr Phe Thr Ser Tyr
             20                  25                  30

Trp Met His Trp Val Lys Gln Ala Pro Gly Gln Gly Leu Glu Trp Ile
         35                  40                  45

Gly Tyr Ile Thr Pro Ser Thr Gly Tyr Thr Asp Tyr Asn Lys Lys Phe
     50                  55                  60

Lys Asp Lys Ala Thr Leu Thr Ala Asp Arg Ser Ser Ser Thr Ala Tyr
65               70                  75                  80

Met Glu Leu Ser Ser Leu Arg Ser Glu Asp Thr Ala Val Tyr Tyr Cys
             85                  90                  95

Ala Arg Ser Gly Pro Tyr Phe Asp Val Trp Gly Ala Gly Thr Thr Val
             100                 105                 110

```
Thr Val Ser Ser
            115


<210>   32
<211>   116
<212>   PRT
<213>   Artificial

<220>
<223>   H chain V region sequence of humanized antibody abb 1782

<400>   32

Gln Val Gln Leu Val Gln Ser Gly Ala Glu Val Lys Lys Pro Gly Ala
1               5                   10                  15


Ser Val Lys Val Ser Cys Lys Ala Ser Gly Tyr Thr Phe Thr Ser Tyr
            20                  25                  30


Trp Met His Trp Val Arg Gln Ala Pro Gly Gln Gly Leu Glu Trp Met
            35                  40                  45


Gly Tyr Ile Thr Pro Ser Thr Gly Tyr Thr Asp Tyr Asn Gln Lys Phe
        50                  55                  60


Gln Gly Arg Val Thr Leu Thr Ala Asp Arg Ser Ser Ser Thr Ala Tyr
65                  70                  75                  80


Met Glu Leu Ser Ser Leu Arg Ser Glu Asp Thr Ala Val Tyr Tyr Cys
                85                  90                  95


Ala Arg Ser Gly Pro Tyr Phe Asp Val Trp Gly Ala Gly Thr Thr Val
            100                 105                 110


Thr Val Ser Ser
            115


<210>   33
<211>   116
<212>   PRT
<213>   Artificial

<220>
<223>   H chain V region sequence of humanized antibody sdr 1782

<400>   33

Gln Val Gln Leu Val Gln Ser Gly Ala Glu Val Lys Lys Pro Gly Ala
1               5                   10                  15


Ser Val Lys Val Ser Cys Lys Ala Ser Gly Tyr Thr Phe Thr Ser Tyr
            20                  25                  30
```

```
Trp Met His Trp Val Lys Gln Ala Pro Gly Gln Gly Leu Glu Trp Ile
        35              40              45

Gly Tyr Ile Thr Pro Ser Thr Gly Tyr Thr Asp Tyr Asn Gln Lys Phe
    50              55              60

Gln Gly Lys Ala Thr Leu Thr Ala Asp Arg Ser Ser Ser Thr Ala Tyr
65              70              75              80

Met Glu Leu Ser Ser Leu Arg Ser Glu Asp Thr Ala Val Tyr Tyr Cys
            85              90              95

Ala Arg Ser Gly Pro Tyr Phe Asp Val Trp Gly Ala Gly Thr Thr Val
            100             105             110

Thr Val Ser Ser
        115


<210>   34
<211>   116
<212>   PRT
<213>   Artificial

<220>
<223>   H chain V region sequence of humanized antibody fra 1782

<400>   34

Gln Val Gln Leu Val Gln Ser Gly Ala Glu Val Lys Lys Pro Gly Ala
1               5               10              15

Ser Val Lys Val Ser Cys Lys Ala Ser Gly Tyr Thr Phe Thr Ser Tyr
            20              25              30

Trp Met His Trp Val Lys Gln Ala Pro Gly Gln Gly Leu Glu Trp Ile
        35              40              45

Gly Tyr Ile Thr Pro Ser Thr Gly Tyr Thr Asp Tyr Asn Lys Lys Phe
    50              55              60

Lys Asp Lys Ala Thr Leu Thr Ala Asp Arg Ser Ser Ser Thr Ala Tyr
65              70              75              80

Met Glu Leu Ser Ser Leu Arg Ser Glu Asp Thr Ala Val Tyr Tyr Cys
            85              90              95

Ala Arg Ser Gly Pro Tyr Phe Asp Val Trp Gly Ala Gly Thr Thr Val
            100             105             110
```

```
Thr Val Ser Ser
       115
```